# EUROPEAN PATENT APPLICATION

(11) **EP 4 741 401 A1**
(43) Date of publication of application: **13.05.2026**
(21) Application number: 24836066.1
(22) Date of filing: 03.07.2024
(51) Int. Cl.: C07D 513/04, A61K 31/429, A61P 3/00, A61P 3/10, A61P 9/00, A61P 13/12, A61P 19/08, A61P 25/00, A61P 35/00, A61P 37/02, A61P 43/00

(54) **BENZOTHIAZOIMIDAZOLYL COMPOUND AND USE THEREOF**

(30) Priority: 05.07.2023 JP 2023110729
(71) Applicant: ER Stress Research Institute, Inc., Tokushima-shi, Tokushima 770-8503 (JP)
(72) Inventor: OYADOMARI, Seiichi, Tokushima-shi, Tokushima 770-8503 (JP)
(74) Representative: Kraus & Lederer PartGmbB
(86) International application number: PCT/JP2024/024053
(87) International publication number: WO 2025/009553

(57) **Abstract**

A compound represented by General Formula (1) is useful for preventing, improving, or treating diseases caused by endoplasmic reticulum stress. 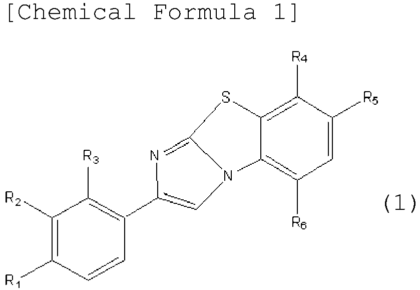 In General Formula (1), R₁ represents a hydrogen atom, a halogen atom, a lower alkyl group, a halogen-substituted lower alkyl group, a lower alkoxy group, a functional group represented by following General Formula (2), or a functional group represented by following General Formula (3), 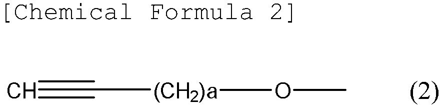 (In Formula (2), a represents an integer of 1-6.) 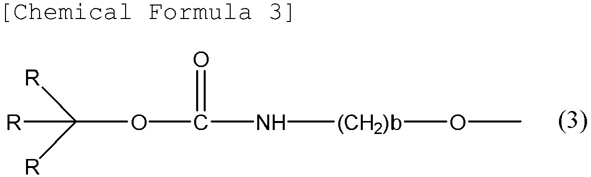 (In Formula (3), b represents an integer of 1-6, and Rs independently represent an alkyl group having 1-6 carbon atoms.)
R₂ represents a hydrogen atom, a halogen atom, a lower alkoxy group, a functional group represented by General Formula (2) above, a functional group represented by following General Formula (4), or a functional group represented by following General Formula (5), 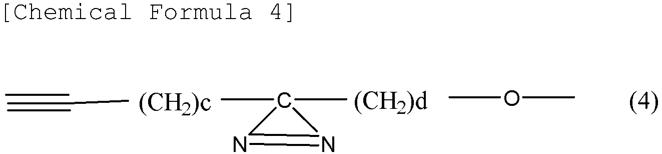 (In Formula (4), c and d independently represent an integer of 1-6.) 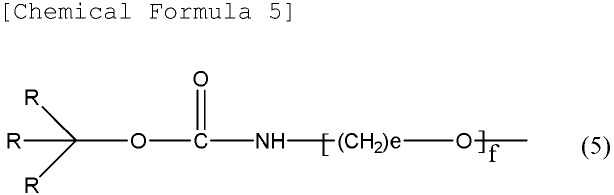 (In Formula (5), e and f independently represent an integer of 1-6, and Rs independently represent an alkyl group having 1-6 carbon atoms.)
R₃ represents a hydrogen atom or a halogen atom,
R₄ represents a hydrogen atom, a halogen atom, a functional group of following General Formula (6), or a functional group of following General Formula (7), 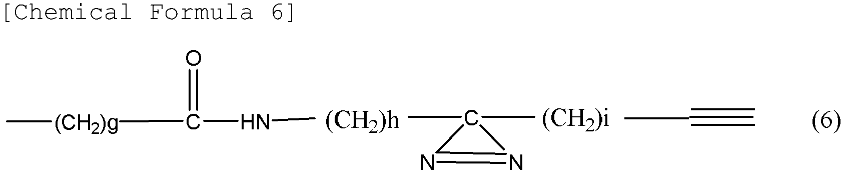 (In Formula (6), g, h, and i independently represent an integer of 1-6.) 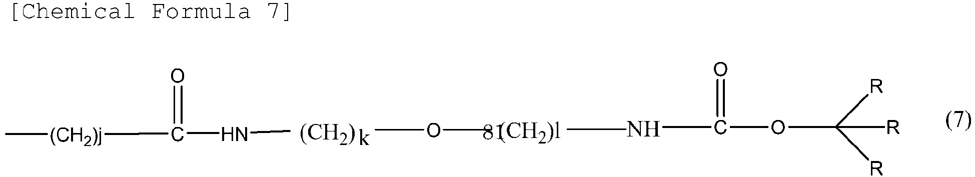 (In Formula (7), j, k, and l independently represent an integer of 1-6, and Rs independently represent an alkyl group having 1-6 carbon atoms.)
R₅ represents a hydrogen atom, a lower alkyl group, a lower alkoxy group, a hydroxy-substituted lower alkyl group, a lower alkyl-substituted sulfonyl group, a lower alkoxy-substituted carbonyl group, a functional group represented by following General Formula (8), a functional group represented by General Formula (9), a functional group represented by General Formula (10), a functional group represented by General Formula (11), a functional group represented by General Formula (12), a functional group represented by General Formula (13), or a functional group represented by General Formula (14), 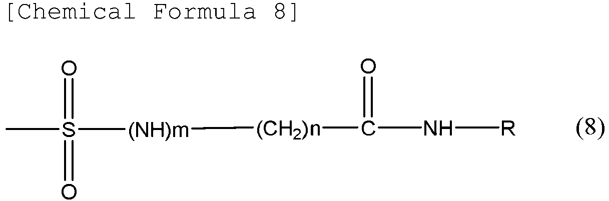 (In Formula (8), m and n independently represent an integer of 1-6, and Rs independently represent an alkyl group having 1-6 carbon atoms.) 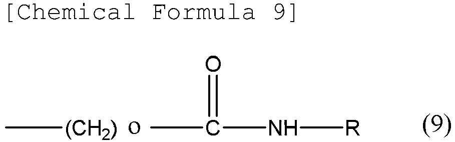 (In Formula (9), o represents an integer of 1-6, and R represents an alkyl group having 1-6 carbon atoms.) 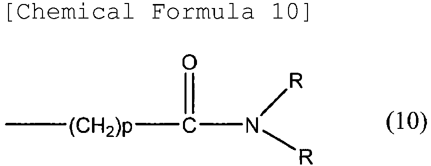 (In Formula (10), p represents an integer of 1-6, and Rs independently represent an alkyl group having 1-6 carbon atoms.) 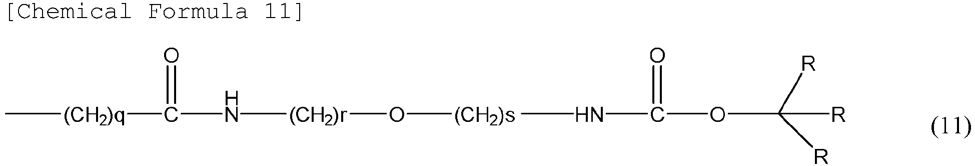 (In Formula (11), q, r, and s independently represent an integer of 1-6, and Rs independently represent an alkyl group having 1-6 carbon atoms.) 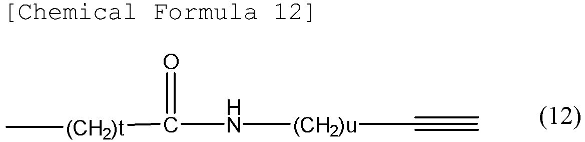 (In Formula (12), t and u independently represent an integer of 1-6.) 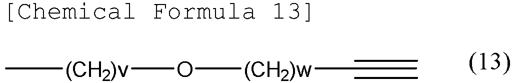 (In Formula (13), v and w independently represent an integer of 1-6.) 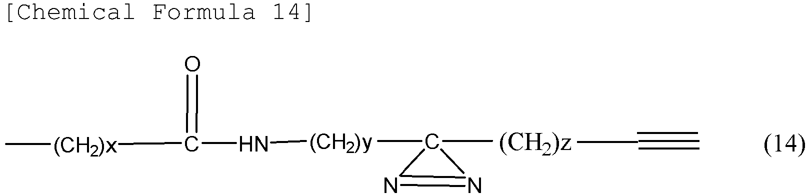 (In Formula (14), x, y, and z independently represent an integer of 1-6.)
R₆ represents a hydrogen atom or a lower alkoxy group.

## Description

### Technical Field

The present invention relates to a benzothiazoimidazolyl compound, a chemical chaperone comprising the compound, a cytoprotectant comprising the compound against endoplasmic reticulum stress, and a method of preventing, improving, or treating a neurodegenerative disease, diabetes mellitus, a metabolic disease, a cardiovascular disease, cancer, a renal disorder, osteodystrophy, a cartilage tissue disease, or an autoimmune disease, using the compound.

### Background Art

The endoplasmic reticulum functions as the site for protein folding and post-translational modification, and about 30% of the protein biosynthesized in cells undergoes modification and maturation in the endoplasmic reticulum. Living organisms are constantly exposed to various stresses from the external environment, and the stresses at the cellular level include defective nutrition (including amino acids, sugars, and lipids), changes in intracellular calcium concentration, hypoxia, heat shock, oxidative stress, virus infection, and mutant protein expression. If cells are exposed to such stress, proteins fail to fold properly in the endoplasmic reticulum lumen, and misfolded proteins accumulate. The state is called endoplasmic reticulum stress.

Upon endoplasmic reticulum stress, cells activate a series of adaptive response mechanisms collectively referred to as the unfolded protein response (UPR), also known as the endoplasmic reticulum stress response, to eliminate aberrant proteins and maintain endoplasmic reticulum function to maintain healthy conditions.

The conditions of the endoplasmic reticulum lumen are sensed by membrane proteins on the endoplasmic reticulum membrane. Of the sensors, IRE1 (inositol requiring enzyme 1), PERK (PKR-like endoplasmic reticulum kinase), and ATF6 (activating transcription factor 6) are expressed non-tissue-specifically. In the steady state, a molecular chaperone BiP/GRP78 binds to these sensor proteins. If misfolded proteins accumulate, BiP/GRP78 dissociates from the sensor proteins and mediates the folding of the misfolded proteins. Meanwhile, the sensor proteins become activated and induce endoplasmic reticulum stress response.

In the endoplasmic reticulum stress response, the translation of new proteins is first suppressed to reduce the load on the endoplasmic reticulum. This is achieved by PERK that phosphorylates eIF2α (eukaryotic initiation factor 2α subunit), which is essential for translation initiation.

PERK also targets a transcriptional regulator ATF4 and promotes the translation of ATF4. If the endoplasmic reticulum stress continues, the transcription of CHOP or GADD34 is induced at the downstream of ATF4. GADD34 functions to dephosphorylate the phosphorylated eIF2α for restoration from the protein translation inhibition. CHOP and GADD34 are involved in the induction of apoptosis under prolonged endoplasmic reticulum stress.

Next, to increase the protein folding capacity in the endoplasmic reticulum, activated ATF6 induces molecular chaperones such as BiP/GRP78 and GRP94, which perform protein folding. The ATF6 also induces transcription of XBP-1 and CHOP.

Such misfolded proteins as above try to refold with the help of molecular chaperones or folding enzymes. Many of the proteins that fail to refold are retrotranslocated to the cytoplasm and degraded by the ubiquitin-proteasome system, which is a process known as endoplasmic reticulum-associated degradation (ERAD) (Non-Patent Literature 1 (Medical science, Vol.55, No.5, October 15, 2004, 416-417)). ERAD is primarily induced when IRE1 selectively splices a transcription factor XBP-1 (X-box-binding protein 1) into the active form.

Cells having defects of endoplasmic reticulum stress that are unable to be solved even by such adaptive responses are removed by apoptosis induced by the expression of a transcription factor CHOP or the like.

If the endoplasmic reticulum stress response fails, proteins are improperly folded, or misfolded proteins further aggregate, resulting in the loss of protein functions essential to maintain life. This causes various diseases. By observing an increase in the expression of factors involved in endoplasmic reticulum stress response, or endoplasmic reticulum stress markers, it has been revealed that endoplasmic reticulum stress or the failure of the endoplasmic reticulum stress response is involved in the onset of various diseases.

Typical examples include neurodegenerative diseases such as Alzheimer's disease, Parkinson's disease, amyotrophic lateral sclerosis (ALS), polyglutamine diseases (such as Huntington's disease), Creutzfeldt-Jakob disease, and central diabetes insipidus (including familial neurogenic diabetes insipidus (FNDI), secondary diabetes insipidus, and idiopathic diabetes insipidus). These diseases share a feature of accumulation of misfolded proteins. Alzheimer's disease develops due to the accumulation of amyloid β and tau proteins; Parkinson's disease develops due to the accumulation of α-synuclein; ALS develops due to the accumulation of mutant superoxide dismutase; Huntington's disease develops due to the accumulation of huntingtin protein; and Creutzfeldt-Jakob disease develops due to the accumulation of abnormal prion (Non-Patent Literature 2 (The Journal of Japanese Biochemical Society, Vol.90, No.1, 51-59, 2018)).

Non-Patent Literature 3 (Biomedical Gerontology, 37(3), 9-16, 2013) also discloses that neurodegenerative diseases share a feature of excess accumulation of structurally abnormal proteins in neurons, and this results from impaired endoplasmic reticulum function that breaks homeostasis to cause endoplasmic reticulum stress. The literature also discloses, as such a neurodegenerative disease, Parkinson's disease, polyglutamine diseases, Alzheimer's disease, and amyotrophic lateral sclerosis.

The familial neurogenic diabetes insipidus (FNDI) will be described in detail. In FNDI, the secretion of an antidiuretic hormone, vasopressin (ADH), decreases, and polyuria and thirst is observed. FNDI is an autosomal dominant disorder, and many causative gene mutations have been reported. FNDI is thought to be caused by endoplasmic reticulum stress resulting from the accumulation of mutant proteins in the endoplasmic reticulum of vasopressin neurons (Non-Patent Literature 4 (The Current Medicine, 63(2):89-92, 2015)).

Non-Patent Literature 5 (Am J Physiol Regul Integr Comp Physiol 296: R1641-R1649, 2009) teaches that in FNDI mice, misfolded proteins accumulate in the endoplasmic reticulum of neurons secreting vasopressin (ADH) (also called AVP) and cause endoplasmic reticulum stress, and the stress induces the cell death of vasopressin neurons to increase the urine output with time.

Non-Patent Literature 6 (Neuroscience Letters 682(2018), 50-55) discloses that oral administration of 4-PBA, a chemical chaperone of reducing endoplasmic reticulum stress, to FNDI model mice reduces the accumulation of mutant proteins in the endoplasmic reticulum, increases the vasopressin secretion, and reduces the urine output.

Endoplasmic reticulum stress can also cause diabetes mellitus.

In Akita mice, the 96th Cys of the protein encoded by the Ins2 gene, one of the insulin genes, mutates to Tyr. This prevents the formation of disulfide bonds, and misfolded proteins accumulate in the endoplasmic reticulum. Accordingly, pancreas β cells disappear, and diabetes mellitus develops (Non-Patent Literature 2 (The Journal of Japanese Biochemical Society, Vol.90, No.1, 2018, 51-59)).

It is also known that PERK knockout mice exhibit a diabetes mellitus-like phenotype and that Wolcott-Rallison syndrome exhibiting insulin-dependent diabetes mellitus symptoms is associated with mutations in the PERK gene (Non-Patent Literature 2 (The Journal of Japanese Biochemical Society, Vol.90, No.1, 2018, 51-59)) .

Not only type 1 diabetes mellitus but also type 2 diabetes mellitus is known to be caused by endoplasmic reticulum stress. For example, Non-Patent Literature 7 (Diabetologia, 50, 752-763, 2007) discloses that endoplasmic reticulum stress markers increase in the pancreas β cells in type 2 diabetic patients as compared with non-diabetic humans. Non-Patent Literature 8 (SCIENCE Vol.313, 25 August, 2006, 1137-1140) discloses that endoplasmic reticulum stress is a major factor associated with obesity, insulin resistance, and type 2 diabetes mellitus and that a chemical chaperone increases the adaptability of endoplasmic reticulum and potentially exerts strong antidiabetic effects in diabetes treatment.

Endoplasmic reticulum stress can also cause metabolic diseases such as obesity, dyslipidemia, and steatohepatitis.

In obese patients, BiP/GRP78 expression increases due to PERK phosphorylation, eIF2α phosphorylation in the PERK pathway, and ATF6 signaling. The IRE1, PERK, and ATF6 pathways are involved in the regulation of lipid metabolism and the progression of steatohepatitis (Non-Patent Literature 2 (The Journal of Japanese Biochemical Society, Vol.90, No.1, 2018, 51-59)).

High dietary fat intake increases the expression of BiP/GRP78, ATF4, CHOP, and the like and also increases the expression of inflammatory response markers such as IL-6 and TNF 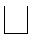 , and macrophages infiltrate adipose tissue. Hence, chronic inflammation occurs in metabolic diseases, and the chronic inflammation is thought to be caused by endoplasmic reticulum stress. In fact, inflammatory response by high dietary fat intake is suppressed by administration of 4-PBA or TUDCA, chemical chaperones that reduce endoplasmic reticulum stress. It is clear that the inflammation caused by endoplasmic reticulum stress can cause metabolic diseases (Non-Patent Literature 2 (The Journal of Japanese Biochemical Society, Vol.90, No.1, 2018, 51-59)).

Non-Patent Literature 3 (Biomedical Gerontology, 37(3), 9-16, 2013) teaches that metabolic diseases such as obesity, inflammatory bowel diseases, and the like are also associated with endoplasmic reticulum stress.

Cardiovascular diseases such as arteriosclerosis and myocardial infarction and cerebral infarction arising from arteriosclerosis are also thought to be primarily caused by chronic inflammation, and thus endoplasmic reticulum stress can also cause these diseases (Non-Patent Literature 2 (The Journal of Japanese Biochemical Society, Vol.90, No.1, 2018, 51-59)).

Non-Patent Literature 9 (DOI:10.1161/CIRCULATIONAHA. 110. 014050) teaches that the progress of arteriosclerosis is affected by the interaction of many biological pathways including endoplasmic reticulum stress. Non-Patent Literature 9 also teaches that CHOP expressed in vascular cells may contribute to vascular remodeling and arteriosclerosis progression.

Non-Patent Literature 3 (Biomedical Gerontology, 37(3), 9-16, 2013) also teaches that cardiac hypertrophy, heart failure, and the like are also associated with endoplasmic reticulum stress.

Endoplasmic reticulum stress can also cause high blood pressure (a cardiovascular disease).

When angiotensin II bonds to type 1 receptor, blood pressure rises. It is known that in angiotensin II-induced hypertensive model mice, the angiotensin II promotes transcription of ATF4 and CHOP to promote phosphorylation of eIF2α and that administrating a chemical chaperone, 4-PBA or TUDCA to the model mice lowers the blood pressure (Non-Patent Literature 2 (The Journal of Japanese Biochemical Society, Vol.90, No.1, 2018, 51-59)).

It is also known that administrating tunicamycin (Tm), which induces endoplasmic reticulum stress, to normal mice increases the blood pressure and that suppressing endoplasmic reticulum stress in spontaneously hypertensive mice lowers the blood pressure and weakens endothelium-dependent vasoconstriction of the aorta (Non-Patent Literature 2 (The Journal of Japanese Biochemical Society, Vol.90, No.1, 2018, 51-59)).

Endoplasmic reticulum stress can also cause various cancers.

Cancer cells obtain nutrients by angiogenesis around the cells. Vascular endothelial growth factor (VEGF) involved in the angiogenesis is induced via the PERK-ATF4 pathway under endoplasmic reticulum stress. PERK and ATF4 induced thereby help epithelial cells to acquire migratory and invasive capabilities and conversion of the cells into mesenchymal cells and also promote the survival and metastasis of cancer cells. XBP-1 activated by IRE1 shows increased expression in breast or hepatocellular cancer. It is thought that the XBP-1 expression induces BiP/GRP78 expression to contribute to the survival of cancer cells (Non-Patent Literature 2 (The Journal of Japanese Biochemical Society, Vol.90, No.1, 2018, 51-59)) .

Non-Patent Literature 10 (The Journal of Clinical Investigation, Vol.125, No.7, July 2015) teaches that integrated stress response (ISR), which includes endoplasmic reticulum stress, is a key mediator of cancer cell survival and that targeting the integrated stress response inhibits tumor progression.

Non-Patent Literature 3 (Biomedical Gerontology, 37(3), 9-16, 2013) also teaches that cancer is associated with endoplasmic reticulum stress.

Endoplasmic reticulum stress can also cause renal disorders.

It is known that in nephritis model rats exhibiting symptoms similar to human membranous nephropathy, eIF2α phosphorylation increases, that in minimal change nephrotic syndrome model rats, BiP/GRP78 expression increases in renal tissue, and that in focal segmental glomerulosclerosis model mice, BiP/GRP78 or GRP94 expression increases, and eIF2α phosphorylation increases. It is known that acute kidney injury model animals are generated by administration of Tm, which causes endoplasmic reticulum stress, and that administration of a chemical chaperone 4-PBA alleviates the symptoms (Non-Patent Literature 2 (The Journal of Japanese Biochemical Society, Vol.90, No.1, 2018, 51-59)).

Endoplasmic reticulum stress can also cause osteodystrophy.

The skeletal system consists of cells (osteoblasts, osteoclasts, osteocytes, and chondrocytes) and extracellular matrices (collagen and hydroxyapatite) produced by cells. Even after growth, cells constantly produce matrix proteins to replace old tissue.

Osteogenesis imperfecta develops due to the absence or dysfunction of endoplasmic reticulum molecular chaperones such as CRTAP, P3H1, CYPB, FKBP65, and HSP47.

Wolcott-Rallison syndrome exhibiting multiple epiphyseal dysplasia or bone loss is known to be caused by PERK gene mutations. In the Wolcott-Rallison, a large amount of collagen accumulates in the endoplasmic reticulum, and the PERK pathway is suggested to be involved in collagen folding.

Coffin-Lowry syndrome exhibits skeletal abnormalities, and the causative gene thereof is the gene of RSK2 that phosphorylates ATF4 (Non-Patent Literature 2 (The Journal of Japanese Biochemical Society, Vol.90, No.1, 2018, 51-59), Non-Patent Literature 3 (Biomedical Gerontology, 37(3), 9-16, 2013)).

Endoplasmic reticulum stress or endoplasmic reticulum stress response can further cause cartilage tissue diseases. In Metaphyseal chondrodysplasia type Schmid (MCDS), the type X collagen gene is mutated, and endoplasmic reticulum stress response is activated in hypertrophic chondrocytes (Non-Patent Literature 3 (Biomedical Gerontology, 37(3), 9-16, 2013)).

Endoplasmic reticulum stress can also cause autoimmune diseases.

For example, Non-Patent Literature 11 (Immunology 225(2020) 151881) teaches that the pathogenesis of autoimmune diseases is often associated with endoplasmic reticulum stress that is caused by accumulation of misfolded proteins.

Accordingly, drug discovery targeting the endoplasmic reticulum stress response has attracted attention. For example, from the search for compounds that prevent neuronal cell death due to endoplasmic reticulum stress, a low-molecular weight compound, salubrinal, has been identified. The compound promotes eIF2α phosphorylation to suppress the synthesis of proteins to be misfolded, thereby reducing the endoplasmic reticulum stress (Non-Patent Literature 12 (Exp Cell Res 314, 950-960(2008)).

Molecular chaperones are proteins that assist in the transport of proteins translated from mRNA transcribed in the nucleus into the endoplasmic reticulum, proper folding of the proteins in the endoplasmic reticulum, and the secretion of the proteins from the endoplasmic reticulum. Molecular chaperones also assist in protein assembly, refolding misfolded proteins, preventing misfolded proteins from aggregating, and endoplasmic reticulum stress-associated degradation (ERAD) and are essential factors for cells to function properly. Hence, it has been thought that if the molecular chaperone network breaks down due to cell senescence or excess stress, many proteins involved in maintaining cell homeostasis become dysfunctional, and various diseases such as neurodegenerative diseases, cardiovascular diseases, diseases arising from abnormal metabolism, and tumor progress (Non-Patent Literature 13 (Journal of Bioscience and Bioengineering, Vol.93, No.4, 2015, 213-215)).

Accordingly, as a therapeutic method considering the alleviation of endoplasmic reticulum stress, administration of a chemical chaperone as a low-molecular weight compound that complements or assists in the function of molecular chaperones (protein chaperones) has been studied.

It has been reported that administration of 4-phenylbutyric acid (4-PBA) or tauroursodeoxycholic acid (TUDCA), which is a modified form thereof, improves pathological conditions including insulin resistance in type 2 diabetes model mice (Non-Patent Literature 8 (SCIENCE 313, 1173-1140(2006)). In addition, trimethylamine N-oxide (TMAO), dimethyl sulfoxide, and ursodeoxycholic acid are also known as the chemical chaperone that improves endoplasmic reticulum functions (Non-Patent Literature 14 (Experimental Medicine, Special issue, Vol.27, No.7, April 10, 2009, Bio Keyword Collection)). Although 4-PBA or TUDCA showed effects only at high doses, azoramide that exhibits antidiabetic effects at a lower concentration has also been reported.

### Citation List

### Non-Patent Documents

[Non-Patent Document 1] Medical science, Vol.55, No.5, October 15, 2004, 416-417,
[Non-Patent Document 2] The Journal of Japanese Biochemical Society, Vol.90, No.1, 2018, 51-59
[Non-Patent Document 3] Biomedical Gerontology, 37(3), 9-16, 2013
[Non-Patent Document 4] The Current Medicine, 63(2):89-92, 2015)
[Non-Patent Document 5] Am J Physiol Regul Integr Comp Physiol 296: R1641-R1649, 2009
[Non-Patent Document 6] Neuroscience Letters 682(2018), 50-55
[Non-Patent Document 7] Diabetologia, 50, 752-763, 2007
[Non-Patent Document 8] SCIENCE 313, 25 August, 2006, 1173-1140
[Non-Patent Document 9] DOI:10.1161/CIRCULATIONAHA. 110. 014050
[Non-Patent Document 10] The Journal of Clinical Investigation, Vol.125, No.7, July 2015
[Non-Patent Document 11] Immunology 225(2020) 151881
[Non-Patent Document 12] Exp Cell Res 314, 950-960(2008)
[Non-Patent Document 13] Journal of Bioscience and Bioengineering, Vol.93, No.4, 2015, 213-215
[Non-Patent Document 14] Experimental Medicine, Special issue, Vol.27, No.7, April 10, 2009, Bio Keyword Collection

### Summary of Invention

### Technical Problem

The objective of the present invention is to provide a novel compound useful for prevention, improvement, or treatment of diseases arising from endoplasmic reticulum stress. The objective of the present invention is also to provide a novel chemical chaperone comprising the compound, a cytoprotectant against endoplasmic reticulum stress comprising the compound, and an agent for prevention, improvement, or treatment of diseases arising from endoplasmic reticulum stress comprising the compound.

### Solution to Problem

The inventor of the present invention has constructed a screening system for compounds that reduce endoplasmic reticulum stress by using endoplasmic reticulum stress response marker genes (JP 2013-187106 A), has found, from about 220,000 compounds, 1,920 2-phenylimidazo[2,1-b]benzothiazole derivatives (IBTs) as chemical chaperones, and has created a lead compound IBT-21 (WO2019/131656A).

IBT-21 is a compound of General Formula (1) in which R₁, R₂, R₃, R₄, and R₆ are each a hydrogen atom, and R₅ is a methylsulfonyl group, and has chemical chaperone activity that alleviates or reduces endoplasmic reticulum stress itself. This has been revealed as follows: IBT-21 suppresses the expression or activation of endoplasmic reticulum stress marker molecules and protects cells from tunicamycin (Tm), a compound of inducing endoplasmic reticulum stress. The chemical chaperone activity of IBT-21 is much stronger than those of known chemical chaperones, 4-PBA, azoramide, and TUDCA. The 50% inhibitory concentration for cell growth inhibition by endoplasmic reticulum stress is 0.61 µM for IBT-21. While on the contrary, it is 5,400 µM for 4-PBA, 86 µM for azoramide, and 2,100 µM for TUDCA (WO2019/131656A).

The inventor of the present invention has searched, from the lead compound, for compounds with stronger chemical chaperone activity and has found compounds represented by General Formula (1). The compound represented by General Formula (1) strongly inhibited the activity of a target molecule of endoplasmic reticulum stress response induced by tunicamycin (Tm). The compound represented by General Formula (1) effectively suppressed cell death by tunicamycin and effectively protected cells from endoplasmic reticulum stress.

The inventor of the present invention has also found that when orally administered, the compound represented by General Formula (1) migrates into the blood and the brain. The inventor has further found that when orally administered to familial neurogenic diabetes insipidus model animals, the compound represented by General Formula (1) reduces the number of mutant protein aggregates in the brain and improves polyuria symptoms. 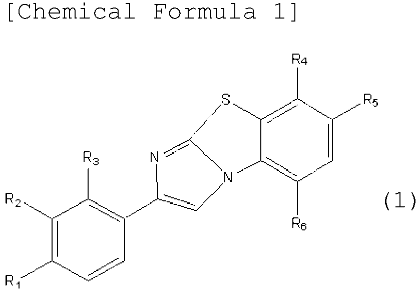 In General Formula (1), R₁ represents a hydrogen atom, a halogen atom, a lower alkyl group, a halogen-substituted lower alkyl group, a lower alkoxy group, a functional group represented by following General Formula (2), or a functional group represented by following General Formula (3), 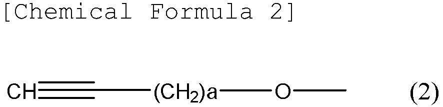 (In Formula (2), a represents an integer of 1-6.) 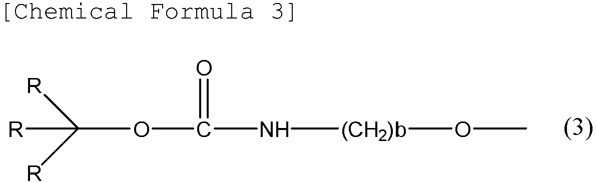 (In Formula (3), b represents an integer of 1-6, Rs independently represent an alkyl group having 1-6 carbon atoms.)
R₂ represents a hydrogen atom, a halogen atom, a lower alkoxy group, a functional group represented by General Formula (2) above, a functional group represented by following General Formula (4), or a functional group represented by following General Formula (5), 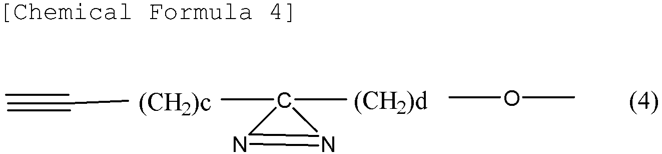 (In Formula (4), c and d independently represent an integer of 1-6.) 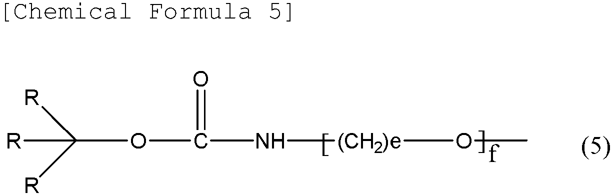 (In Formula (5), e and f independently represent an integer of 1-6, and Rs independently represent an alkyl group having 1-6 carbon atoms.)
R₃ represents a hydrogen atom or a halogen atom,
R₄ represents a hydrogen atom, a halogen atom, a functional group of following General Formula (6), or a functional group of following General Formula (7), 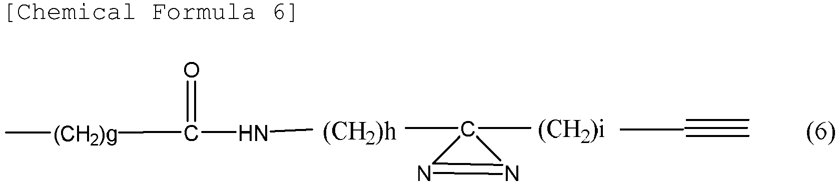 (In Formula (6), g, h, and i independently represent an integer of 1-6.) 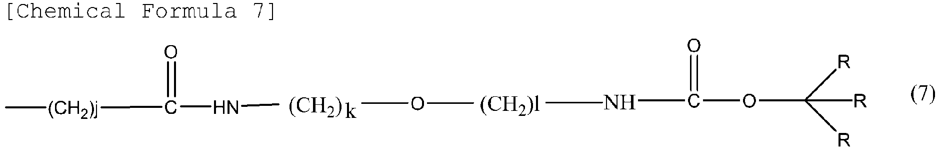 (In Formula (7), j, k, and l independently represent an integer of 1-6, and Rs independently represent an alkyl group having 1-6 carbon atoms.)
R₅ represents a hydrogen atom, a lower alkyl group, a lower alkoxy group, a hydroxy-substituted lower alkyl group, a lower alkyl-substituted sulfonyl group, a lower alkoxy-substituted carbonyl group, a functional group represented by following General Formula (8), a functional group represented by General Formula (9), a functional group represented by General Formula (10), a functional group represented by General Formula (11), a functional group represented by General Formula (12), a functional group represented by General Formula (13), or a functional group represented by General Formula (14), 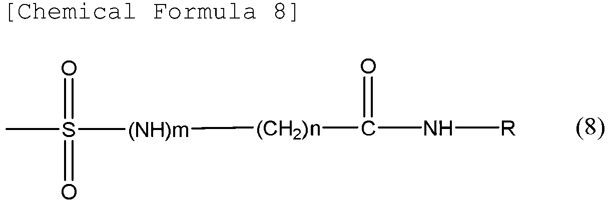 (In Formula (8), m and n independently represent an integer of 1-6, and Rs independently represent an alkyl group having 1-6 carbon atoms.) 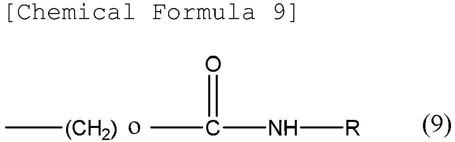 (In Formula (9), o represents an integer of 1-6, and R represents an alkyl group having 1-6 carbon atoms.) 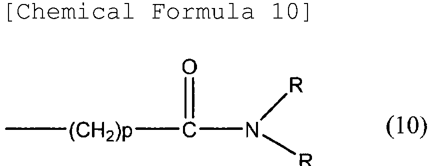 (In Formula (10), p represents an integer of 1-6, and Rs independently represent an alkyl group having 1-6 carbon atoms.) 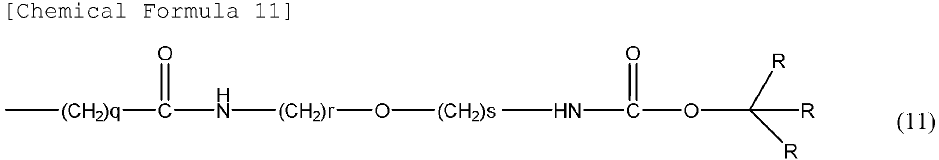 (In Formula (11), q, r, and s independently represent an integer of 1-6, and Rs independently represent an alkyl group having 1-6 carbon atoms.) 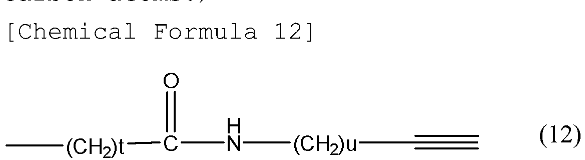 (In Formula (12), t and u independently represent an integer of 1-6.) 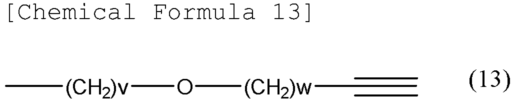 (In Formula (13), v and w independently represent an integer of 1-6.) 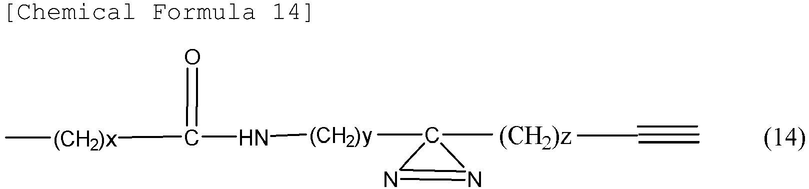 (In Formula (14), x, y, and z independently represent an integer of 1-6.)
R₆ represents a hydrogen atom or a lower alkoxy group.

[1] A compound represented by General Formula (1). 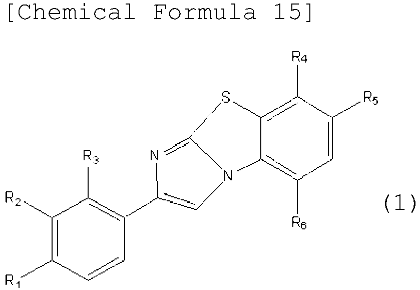 In General Formula (1), R₁ represents a hydrogen atom, a halogen atom, a lower alkyl group, a halogen-substituted lower alkyl group, a lower alkoxy group, a functional group represented by following General Formula (2), or a functional group represented by following General Formula (3), 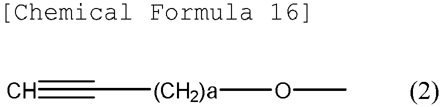 (In Formula (2), a represents an integer of 1-6.) 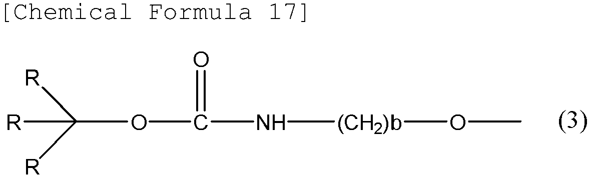 (In Formula (3), b represents an integer of 1-6, and Rs independently represent an alkyl group having 1-6 carbon atoms.)
   R₂ represents a hydrogen atom, a halogen atom, a lower alkoxy group, a functional group represented by General Formula (2) above, a functional group represented by following General Formula (4), or a functional group represented by following General Formula (5), 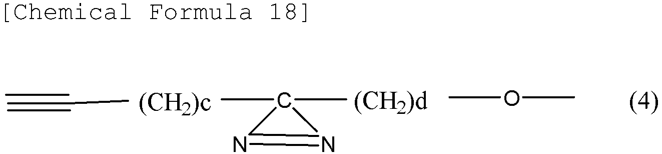 (In Formula (4), c and d independently represent an integer of 1-6.) 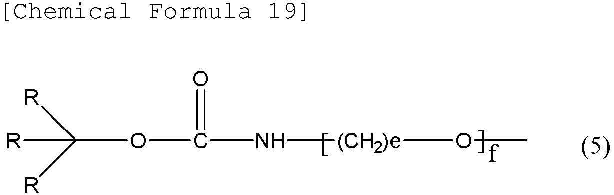 (In Formula (5), e and f independently represent an integer of 1-6, and Rs independently represent an alkyl group having 1-6 carbon atoms.)
   R₃ represents a hydrogen atom or a halogen atom,
   R₄ represents a hydrogen atom, a halogen atom, a functional group of following General Formula (6), or a functional group of following General Formula (7), 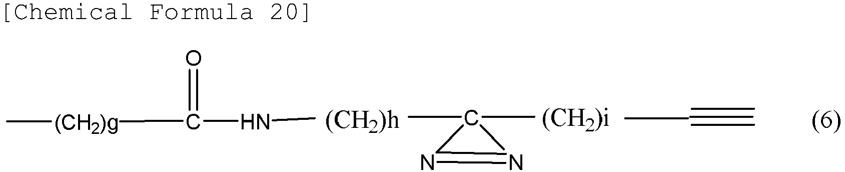 (In Formula (6), g, h, and i independently represent an integer of 1-6.) 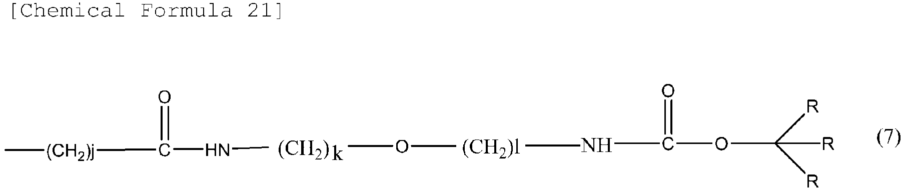 (In Formula (7), j, k, and l independently represent an integer of 1-6, and Rs independently represent an alkyl group having 1-6 carbon atoms.)
   R₅ represents a hydrogen atom, a lower alkyl group, a lower alkoxy group, a hydroxy-substituted lower alkyl group, a lower alkyl-substituted sulfonyl group, a lower alkoxy-substituted carbonyl group, a functional group represented by following General Formula (8), a functional group represented by General Formula (9), a functional group represented by General Formula (10), a functional group represented by General Formula (11), a functional group represented by General Formula (12), a functional group represented by General Formula (13), or a functional group represented by General Formula (14), 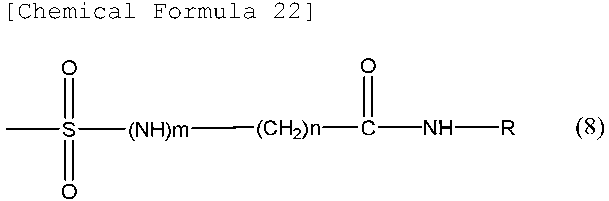 (In Formula (8), m and n independently represent an integer of 1-6, and Rs independently represent an alkyl group having 1-6 carbon atoms.) 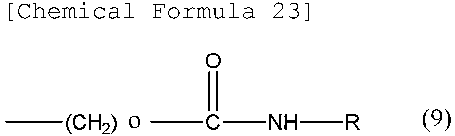 (In Formula (9), o represents an integer of 1-6, and R represents an alkyl group having 1-6 carbon atoms.) 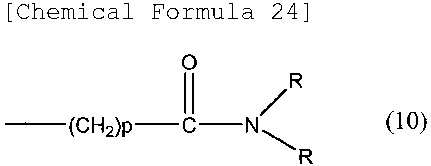 (In Formula (10), p represents an integer of 1-6, and Rs independently represent an alkyl group having 1-6 carbon atoms.) 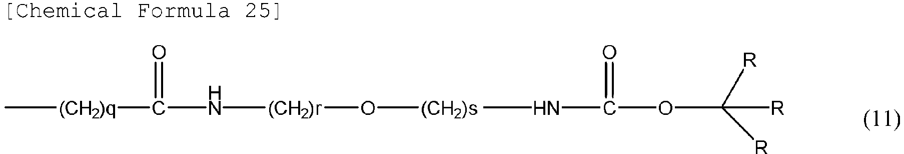 (In Formula (11), q, r, and s independently represent an integer of 1-6, and Rs independently represent an alkyl group having 1-6 carbon atoms.) 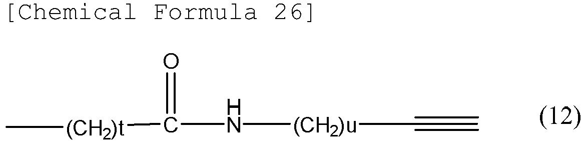 (In Formula (12), t and u independently represent an integer of 1-6.) 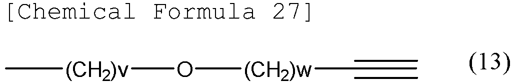 (In Formula (13), v and w independently represent an integer of 1-6.) 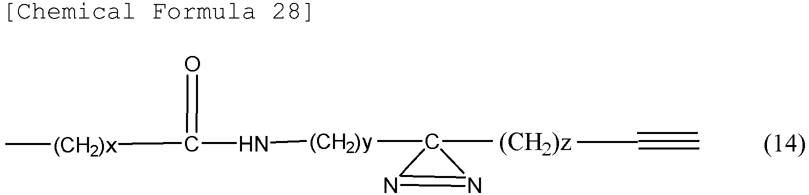 (In Formula (14), x, y, and z independently represent an integer of 1-6.)
   R₆ represents a hydrogen atom or a lower alkoxy group.
[2] The compound according to [1], wherein R₁ is a hydrogen atom, a halogen atom (particularly a chlorine atom), a lower alkyl group (particularly a propyl group), a lower alkoxy group (particularly a methoxy group), a halogen-substituted lower alkyl group (particularly a fluorine-substituted lower alkyl group, specifically a trifluoromethyl group), or a functional group represented by General Formula (2) (particularly a functional group of General Formula (2) in which a is 1),
   R₂ is a hydrogen atom, a halogen atom (particularly a chlorine atom), a lower alkoxy group (particularly a methoxy group), a functional group represented by General Formula (2) (particularly a functional group of General Formula (2) in which a is 1), or a functional group represented by General Formula (4) (particularly a functional group of General Formula (4) in which c and d are each 2),
   R₃ is a hydrogen atom or a chlorine atom,
   R₄ is a hydrogen atom,
   R₅ is a hydrogen atom, a lower alkyl group (particularly a methyl group), a lower alkoxy group (particularly an ethoxy group), a hydroxy-substituted lower alkyl group (particularly a hydroxy-substituted methyl group), a lower alkyl-substituted sulfonyl group (particularly a methyl-substituted sulfonyl group), a lower alkoxy-substituted carbonyl group (particularly an ethoxy-substituted carbonyl group), a functional group represented by General Formula (8) (particularly a functional group of General Formula (8) in which m is 0 or 1, n is 3, and R is a methyl group), a functional group represented by General Formula (9) (particularly a functional group of General Formula (9) in which o is 2, and R is a methyl group), or a functional group represented by General Formula (10) (particularly a functional group of General Formula (10) in which p is 2, and two Rs are each an ethyl group), and
   R₆ is a hydrogen atom or a methoxy group.
[3] The compound according to any one of [1] to [3], wherein R₁ is a halogen-substituted lower alkyl group, R₂ is a lower alkoxy group, R₃ is a hydrogen atom, R₄ is a hydrogen atom, R₅ is a lower alkyl-substituted sulfonyl group, and R₆ is a hydrogen atom;
   a compound in which R₁ is a halogen-substituted lower alkyl group,
   R₂ is a hydrogen atom, R₃ is a hydrogen atom, R₄ is a hydrogen atom,
   R₅ is a lower alkyl-substituted sulfonyl group, and R₆ is a hydrogen atom;
   a compound in which R₁ is a halogen atom, R₂ is a halogen atom, R₃ is a hydrogen atom, R₄ is a hydrogen atom, R₅ is a lower alkyl-substituted sulfonyl group, and R₆ is a hydrogen atom;
   a compound in which R₁ is a hydrogen atom, R₂ is a lower alkoxy group, R₃ is a hydrogen atom, R₄ is a hydrogen atom, R₅ is a lower alkyl-substituted sulfonyl group, and R₆ is a hydrogen atom;
   a compound in which R₁ is a halogen atom, R₂ is a hydrogen atom, R₃ is a hydrogen atom, R₄ is a hydrogen atom, R₅ is a lower alkyl-substituted sulfonyl group, and R₆ is a hydrogen atom;
   a compound in which R₁ is a halogen atom, R₂ is a hydrogen atom, R₃ is a hydrogen atom, R₄ is a hydrogen atom, R₅ is a functional group of General Formula (9) in which o is 2, and R is a methyl group,
   and R₆ is a hydrogen atom;
   a compound in which R₁ is a halogen atom, R₂ is a lower alkoxy group, R₃ is a hydrogen atom, R₄ is a hydrogen atom, R₅ is a lower alkyl-substituted sulfonyl group, and R₆ is a hydrogen atom;
   a compound in which R₁ is a halogen-substituted lower alkyl group,
   R₂ is a hydrogen atom, R₃ is a hydrogen atom, R₄ is a hydrogen atom,
   R₅ is a functional group of General Formula (9) in which o is 2, and R is a methyl group, and R₆ is a hydrogen atom;
   a compound in which R₁ is a halogen-substituted lower alkyl group,
   R₂ is a hydrogen atom, R₃ is a hydrogen atom, R₄ is a hydrogen atom,
   R₅ is a functional group of General Formula (8) in which m is 0, n is 3, and R is a methyl group, and R₆ is a hydrogen atom;
   a compound in which R₁ is a halogen-substituted lower alkyl group,
   R₂ is a hydrogen atom, R₃ is a hydrogen atom, R₄ is a hydrogen atom,
   R₅ is a functional group of General Formula (10) in which p is 2, and two Rs are each an ethyl group, and R₆ is a hydrogen atom;
   a compound in which R₁ is a halogen-substituted lower alkyl group,
   R₂ is a hydrogen atom, R₃ is a hydrogen atom, R₄ is a hydrogen atom,
   R₅ is a hydrogen atom, and R₆ is a hydrogen atom;
   a compound in which R₁ is a functional group of General Formula (2) in which a is 1, R₂ is a hydrogen atom, R₃ is a hydrogen atom, R₄ is a hydrogen atom, R₅ is a lower alkyl-substituted sulfonyl group, and R₆ is a hydrogen atom;
   a compound in which R₁ is a lower alkoxy group, R₂ is a hydrogen atom, R₃ is a hydrogen atom, R₄ is a hydrogen atom, R₅ is a lower alkyl-substituted sulfonyl group, and R₆ is a hydrogen atom;
   a compound in which R₁ is a halogen atom, R₂ is a hydrogen atom, R₃ is a halogen atom, R₄ is a hydrogen atom, R₅ is a lower alkyl-substituted sulfonyl group, and R₆ is a hydrogen atom;
   a compound in which R₁ is a halogen atom, R₂ is a hydrogen atom, R₃ is a hydrogen atom, R₄ is a hydrogen atom, R₅ is a lower alkoxy-substituted carbonyl group, and R₆ is a hydrogen atom;
   a compound in which R₁ is a halogen atom, R₂ is a hydrogen atom, R₃ is a hydrogen atom, R₄ is a hydrogen atom, R₅ is a lower alkoxy group, and R₆ is a hydrogen atom;
   a compound in which R₁ is a lower alkyl-substituted sulfonyl group,
   R₂ is a hydrogen atom, R₃ is a hydrogen atom, R₄ is a hydrogen atom,
   R₅ is a functional group of General Formula (8) in which m is 1, n is 3, and R is a methyl group, and R₆ is a hydrogen atom;
   a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
   a compound in which R₁ is a halogen atom, R₂ is a hydrogen atom, R₃ is a hydrogen atom, R₄ is a hydrogen atom, R₅ is a hydroxy-substituted lower alkyl group, and R₆ is a hydrogen atom;
   a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
   a compound in which R₁ is a halogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom;
   a compound in which R₁ is a lower alkyl group, R₂ is a hydrogen atom, R₃ is a hydrogen atom, R₄ is a hydrogen atom, R₅ is a lower alkyl-substituted sulfonyl group, and R₆ is a hydrogen atom;
   a compound in which R₁ is a lower alkyl-substituted sulfonyl group, R₂ is a hydrogen atom, R₃ is a hydrogen atom, R₄ is a hydrogen atom, R₅ is a lower alkyl group, and R₆ is a hydrogen atom; or
   a compound in which R₁ is a hydrogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom.
[4] The compound according to any one of [1] to [3], wherein R₁ is a hydrogen atom, a fluorine atom, a chlorine atom, a propyl group (particularly, an n-propyl group), a trifluoromethyl group, a methoxy group, an ethoxy group, a functional group of General Formula (2) in which a is 1, or a functional group of General Formula (3) in which b is 3 and the three Rs are methyl group;
   R₂ is a hydrogen atom, a chlorine atom, a fluorine atom, a methoxy group, a functional group of General Formula (2) in which a is 1, a functional group of General Formula (4) in which c and d are each 2, a functional group of General Formula (5) in which e is 2, f is 1, and three Rs are methyl group, or a functional group of General Formula (5) in which e is 2, f is 3, and three Rs are methyl group;
   R₃ is a hydrogen atom or a chlorine atom;
   R₄ is a hydrogen atom, a bromine atom, a functional group of General Formula (6) in which g, h, and i are each 2, or a functional group of General Formula (7) in which j, k, and l are each 2 and three Rs are methyl group;
   R₅ is a hydrogen atom, a methyl group, a methoxy group, a hydroxymethyl group, a methylsulfonyl group, a lower alkyl-substituted oxycarbonyl group, a functional group of General Formula (8) in which m is 0, n is 3, and R is a methyl group, a functional group of General Formula (8) in which m is 1, n is 3, and R is a methyl group, a functional group of General Formula (9) in which o is 2 and R is a methyl group, a functional group of General Formula (10) in which p is 1 and two R groups are ethyl group, a functional group of General Formula (11) in which q, r, and s are each 2 and three Rs are methyl group, a functional group of General Formula (12) in which t and u are each 2, a functional group of General Formula (13) in which v and w are each 2, or a functional group of General Formula (14) in which x, y, and z are each 2;
   R₆ is a hydrogen atom or a methoxy group.
[5] A chemical chaperone comprising the compound according to any one of [1] to [4].
[6] A cytoprotectant against endoplasmic reticulum stress comprising the compound according to any one of [1] to [4].
[7] An agent for alleviating or reducing endoplasmic reticulum stress comprising the compound according to any one of [1] to [4].
[8] an agent for preventing, improving, or treating a disease caused by endoplasmic reticulum stress comprising the compound according to any one of [1] to [4].
[9] The preventing, improving, or treating agent according to [8], wherein the disease caused by endoplasmic reticulum stress is neurodegenerative diseases, diabetes mellitus, metabolic diseases, cardiovascular diseases, cancers, renal disorders, osteodystrophy, cartilage tissue diseases, or autoimmune diseases.

### Advantageous Effects of Invention

The inventor of the present invention has found that endoplasmic reticulum stress response activated by the action of tunicamycin on cells is suppressed by compounds having a benzothiazoimidazolyl skeleton. The inventor has also found that these compounds having a benzothiazoimidazolyl skeleton protect cells from the toxicity caused by endoplasmic reticulum stress induced by tunicamycin and improve the cell viability (WO2019/131656A). If the progress of endoplasmic reticulum stress response is suppressed after endoplasmic reticulum stress occurs, cell dysfunction increases, and cytoprotection is not achieved. This means that the compound having a benzothiazoimidazolyl skeleton alleviates or reduces the endoplasmic reticulum stress itself. Accordingly, the compound of the present invention having a benzothiazoimidazolyl skeleton is rationally predicted to function as a chemical chaperone that alleviates or reduces the endoplasmic reticulum stress itself. In fact, as described in EXAMPLES in the description of the present application, the compound represented by General Formula (1) strongly suppresses endoplasmic reticulum stress response activated by the action of tunicamycin on cells.

As described in EXAMPLES in the description, the compound represented by General Formula (1) strongly suppresses cell growth inhibition caused by endoplasmic reticulum stress induced by tunicamycin. The compound concentration of suppressing cell death by endoplasmic reticulum stress by 50% (IC₅₀) is about 10 □m or less, which is noticeably smaller than the IC₅₀ of azoramide or TUDCA, known chemical chaperones. The compound represented by General Formula (1) strongly protects cells from endoplasmic reticulum stress, and this is thought to be based on the strong chemical chaperone activity.

The compound represented by General Formula (1), which protects cells from endoplasmic reticulum stress, is useful as the component of agents for preventing, improving, or treating diseases caused by endoplasmic reticulum stress or endoplasmic reticulum stress response. Examples of the disease include, as described above, neurodegenerative diseases such as Alzheimer's disease, Parkinson's disease, amyotrophic lateral sclerosis (ALS), polyglutamine diseases (including Huntington's disease), Creutzfeldt-Jakob disease, and central diabetes insipidus (familial neurogenic diabetes insipidus (FNDI), secondary diabetes insipidus, idiopathic diabetes insipidus); metabolic diseases such as diabetes mellitus (type 1 diabetes mellitus, type 2 diabetes mellitus), obesity, dyslipidemia, and steatohepatitis; cardiovascular diseases such as arteriosclerosis, myocardial infarction, cerebral infarction, and high blood pressure; cancers; renal disorders; osteodystrophy; cartilage tissue diseases; and autoimmune diseases.

EXAMPLES in the present description demonstrate that oral administration of IBT-30, a typical example of the compound represented by General Formula (1), to familial neurogenic diabetes insipidus model mice reduced the number of mutant protein aggregates in the brain and suppressed an increase in urine output. Diabetes insipidus includes, in addition to familial neurogenic diabetes insipidus, secondary diabetes insipidus caused by brain tumor, external injury, or the like and idiopathic diabetes insipidus by unknown cause. Vasopressin neurons are vulnerable to endoplasmic reticulum stress, and thus the compound represented by General Formula (1), which protects cells from endoplasmic reticulum stress, is thought to be also useful for improving secondary diabetes insipidus or idiopathic diabetes insipidus.

When orally administered, the compound represented by General Formula (1) migrated into the brain and protected cells from endoplasmic reticulum stress, and thus oral administration thereof is also expected to be useful for improving other neuropsychiatric diseases caused by endoplasmic reticulum stress.

### Brief Description of Drawings

Fig. 1 is a figure illustrating the system for searching for substances that suppress the activity of a target molecule of endoplasmic reticulum stress response induced by tunicamycin.
Fig. 2 is a graph showing decreases in urine output by oral administration of IBT-30, the compound of the present invention, to familial neurogenic diabetes insipidus (FNDI) model mice.
Fig. 3 is a graph showing a decrease in the number of inclusion bodies (mutant protein aggregates) by oral administration of IBT-30, the compound of the present invention, where the number of inclusion bodies (mutant protein aggregates) not smaller than 4.5 µm was counted in the vasopressin neuron region of frozen sections of the suprachiasmatic nucleus in the hypothalamus in the brain of familial neurogenic diabetes insipidus (FNDI) model mice.

### Description of Embodiments

### (1) Compound of the present invention

The compound of the present invention is a benzothiazoimidazolyl compound represented by General Formula (1).

In the present invention, the term "lower" represents a carbon number of 1 to 6 unless otherwise noted and may be a carbon number of 1, 2, 3, 4, 5, or 6.

A halogen atom represents a fluorine atom, a chlorine atom, or a bromine atom.

A halogen-substituted lower alkyl group may be substituted with one, two, or three halogen atoms. The lower alkyl group in this case is an alkyl group having a carbon number of 1 to 5 (1, 2, 3, 4, or 5).

A hydroxy-substituted lower alkyl group may be substituted with one, two, or three hydroxy groups. The lower alkyl group in this case is an alkyl group having a carbon number of 1 to 5 (1, 2, 3, 4, or 5).

A lower alkyl-substituted sulfonyl group may be substituted with one lower alkyl group.

Preferred examples of the R₁ include a hydrogen atom, a halogen atom (particularly a chlorine atom), a lower alkyl group (particularly a propyl group), a lower alkoxy group (particularly a methoxy group), a halogen-substituted lower alkyl group (particularly a fluorine-substituted lower alkyl group, specifically a trifluoromethyl group), and a functional group represented by General Formula (2) (particularly a functional group of General Formula (2) in which a is 1). Particularly preferred examples of the R₁ include a trifluoromethyl group, a fluorine atom, a chlorine atom, and a functional group of General Formula (2) in which a is 1.

Preferred examples of the R₁ include a hydrogen atom, a fluorine atom, a chlorine atom, a propyl group (particularly an n-propyl group), a trifluoromethyl group, a methoxy group, an ethoxy group, a functional group of General Formula (2) in which a is 1, and a functional group of General Formula (3) in which b is 3, and three Rs are each a methyl group.

Preferred examples of the R₂ include a hydrogen atom, a halogen atom (particularly a chlorine atom), a lower alkoxy group (particularly a methoxy group), a functional group represented by General Formula (2) (particularly a functional group of General Formula (2) in which a is 1), and a functional group represented by General Formula (4) (particularly a functional group of General Formula (4) in which c and d are each 2). Particularly preferred examples of the R₂ include a hydrogen atom, a methoxy group, and a chlorine atom.

Preferred examples of the R₂ include a hydrogen atom, a chlorine atom, a fluorine atom, a methoxy group, a functional group of General Formula (2) in which a is 1, a functional group of General Formula (4) in which c and d are each 2, a functional group of General Formula (5) in which e is 3, f is 1, and three Rs are each a methyl group, and a functional group of General Formula (5) in which e is 2, f is 3, and three Rs are each a methyl group.

Preferred examples of the R₃ include a hydrogen atom and a chlorine atom.

Preferred examples of the R₄ include a hydrogen atom, a bromine atom, a functional group of General Formula (6) in which g, h, and i are each 2, and a functional group of General Formula (7) in which j, k, and l are each 2, and three Rs are each a methyl group. Specifically, a hydrogen atom is particularly preferred.

Preferred examples of the R₅ include a hydrogen atom, a lower alkyl group (particularly a methyl group), a lower alkoxy group (particularly an ethoxy group), a hydroxy-substituted lower alkyl group (particularly a hydroxy-substituted methyl group), a lower alkyl-substituted sulfonyl group (particularly a methyl-substituted sulfonyl group), a lower alkoxy-substituted carbonyl group (particularly an ethoxy-substituted carbonyl group), a functional group represented by General Formula (8) (particularly a functional group of General Formula (8) in which m is 0 or 1, n is 3, and R is a methyl group), a functional group represented by General Formula (9) (particularly a functional group of General Formula (9) in which o is 2, and R is a methyl group), and a functional group represented by General Formula (10) (particularly a functional group of General Formula (10) in which p is 2, and two Rs are each an ethyl group). Particularly preferred examples of the R₅ include a methyl-substituted sulfonyl group, a functional group of General Formula (9) in which o is 2, and R is a methyl group, and a functional group of General Formula (8) in which m is 0, n is 3, and R is a methyl group.

Preferred examples of the R₅ include a hydrogen atom, a methyl group, a methoxy group, a hydroxymethyl group, a methylsulfonyl group, an ethyl-substituted oxycarbonyl group, a functional group of General Formula (8) in which m is 0, n is 3, and R is a methyl group, a functional group of General Formula (8) in which m is 1, n is 3, and R is a methyl group, a functional group of General Formula (9) in which o is 2. and R is a methyl group, a functional group of General Formula (10) in which p is 1, and two Rs are each an ethyl group, a functional group of General Formula (11) in which q, r, and s are each 2, and three Rs are each a methyl group, a functional group of General Formula (12) in which t and u are each 2, a functional group of General Formula (13) in which v and w are each 2, and a functional group of General Formula (14) in which x, y, and z are each 2.

Preferred examples of the R₆ include a hydrogen atom and a methoxy group. Specifically, a hydrogen atom is particularly preferred.

Preferred examples of the compound include a compound represented by Formula (1) in which R₁ is a hydrogen atom, a halogen atom (particularly a chlorine atom), a lower alkyl group (particularly a propyl group), a lower alkoxy group (particularly a methoxy group), a halogen-substituted lower alkyl group (particularly a fluorine-substituted lower alkyl group, specifically a trifluoromethyl group), or a functional group represented by General Formula (2) (particularly a functional group of General Formula (2) in which a is 1),
R₂ is a hydrogen atom, a halogen atom (particularly a chlorine atom), a lower alkoxy group (particularly a methoxy group), a functional group represented by General Formula (2) (particularly a functional group of General Formula (2) in which a is 1), or a functional group represented by General Formula (4) (particularly a functional group of General Formula (4) in which c and d are each 2),
R₃ is a hydrogen atom or a chlorine atom,
R₄ is a hydrogen atom,
R₅ is a hydrogen atom, a lower alkyl group (particularly a methyl group), a lower alkoxy group (particularly an ethoxy group), a hydroxy-substituted lower alkyl group (particularly a hydroxy-substituted methyl group), a lower alkyl-substituted sulfonyl group (particularly a methyl-substituted sulfonyl group), a lower alkoxy-substituted carbonyl group (particularly an ethoxy-substituted carbonyl group), a functional group represented by General Formula (8) (particularly a functional group of General Formula (8) in which m is 0 or 1, n is 3, and R is a methyl group), a functional group represented by General Formula (9) (particularly a functional group of General Formula (9) in which o is 2, and R is a methyl group), or a functional group represented by General Formula (10) (particularly a functional group of General Formula (10) in which p is 2, and two Rs are each an ethyl group), and
R₆ is a hydrogen atom or a methoxy group.

Preferred examples of the compound include a compound in which R₁ is a halogen-substituted lower alkyl group, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a lower alkyl-substituted sulfonyl group, and R₆ is a hydrogen atom;
a compound in which R₁ is a halogen atom, R₂ is a halogen atom, R₃ is a hydrogen atom, R₄ is a hydrogen atom, R₅ is a lower alkyl-substituted sulfonyl group, and R₆ is a hydrogen atom;
a compound in which R₁ is a hydrogen atom, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a functional group of General Formula (9) in which o is 2, and R is a methyl group, and R₆ is a hydrogen atom;
a compound in which R₁ is a halogen atom, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (9) in which o is 2, and R is a methyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (8) in which m is 0, n is 3, and R is a methyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (10) in which p is 2, and two Rs are each an ethyl group, and R₆ is a hydrogen atom;
a compound in which R₁ is a halogen-substituted lower alkyl group,
R₂ is a hydrogen atom, R₃ is a hydrogen atom, R₄ is a hydrogen atom, R₅ is a hydrogen atom, and R₆ is a hydrogen atom;
a compound in which R₁ is a functional group of General Formula (2) in which a is 1, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a lower alkoxy group, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a halogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkoxy-substituted carbonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkoxy group, and R₆ is a hydrogen atom;
a compound in which R₁ is a lower alkyl-substituted sulfonyl group, R₂ is a hydrogen atom, R₃ is a hydrogen atom, R₄ is a hydrogen atom, R₅ is a functional group of General Formula (8) in which m is 1, n is 3, and R is a methyl group, and R₆ is a hydrogen atom;
a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a hydroxy-substituted lower alkyl group, and R₆ is a hydrogen atom;
a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom;
a compound in which R₁ is a lower alkyl group, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a lower alkyl-substituted sulfonyl group, R₂ is a hydrogen atom, R₃ is a hydrogen atom, R₄ is a hydrogen atom, R₅ is a lower alkyl group, and R₆ is a hydrogen atom; and
a compound in which R₁ is a hydrogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom.

The compound represented by General Formula (1) of the present invention may be in the form of an acid addition salt or a base addition salt.

The compound represented by General Formula (1) of the present invention can be synthesized, for example, in the following manner.

The carboxyl group of a benzoic acid derivative 1 is methylated with methyllithium to give an acetophenone derivative 2. The acetophenone derivative 2 is next reacted with a quaternary ammonium bromide to give a bromoacetophenone derivative 3.

Separately, an aniline derivative 5' is reacted with ammonium isothiocyanate in the presence of bromine to give an aminobenzothiazole derivative 5, which is subjected to condensation ring closure reaction with the bromoacetophenone derivative 3 in the presence of a base (for example, potassium carbonate) to give a 2-phenyl-benzothiazoimidazole derivative 4.

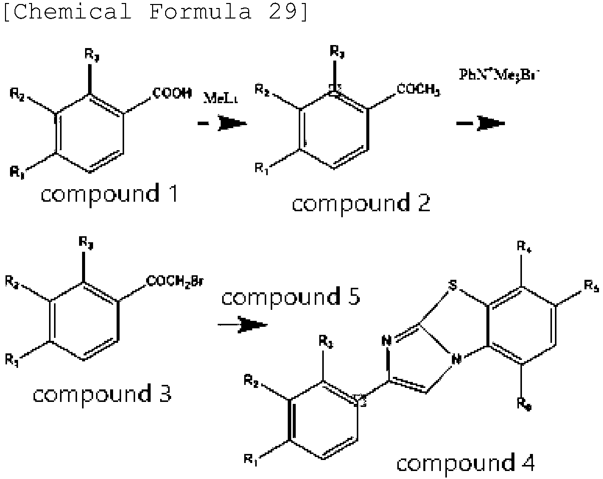

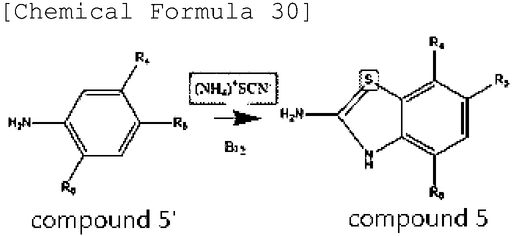

### (2) Use of compound

The compound of the present invention represented by General Formula (1) may be used to protect cells from endoplasmic reticulum stress. Protecting cells from endoplasmic reticulum stress includes a method of alleviating or reducing endoplasmic reticulum stress itself when the endoplasmic reticulum stress is induced (a method of alleviating or reducing endoplasmic reticulum stress before occurrence of the endoplasmic reticulum stress response), and a method of promoting the endoplasmic reticulum stress response to alleviate or reduce cell dysfunction caused by endoplasmic reticulum stress. The compound of the present invention may be used to protect cells from endoplasmic reticulum stress by alleviating or reducing the endoplasmic reticulum stress itself. Specifically, the compound may be used to suppress cell death by endoplasmic reticulum stress (to suppress a decrease in cell viability).

Hence, the compound of the present invention may be used as a chemical chaperone to alleviate or reduce endoplasmic reticulum stress itself. Specifically, the compound may be used to assist in the transport of proteins into the endoplasmic reticulum, the normal folding of proteins in the endoplasmic reticulum, the secretion of normally folded proteins from the endoplasmic reticulum, protein assembly, refolding of misfolded proteins, preventing misfolded proteins from aggregating, and endoplasmic reticulum stress-associated degradation (ERAD).

Examples of the disease caused by endoplasmic reticulum stress include neurodegenerative diseases such as Alzheimer's disease, Parkinson's disease, amyotrophic lateral sclerosis (ALS), polyglutamine diseases (including Huntington's disease), Creutzfeldt-Jakob disease, and central diabetes insipidus (familial neurogenic diabetes insipidus (FNDI), secondary diabetes insipidus, idiopathic diabetes insipidus); metabolic diseases such as diabetes mellitus (type 1 diabetes mellitus, type 2 diabetes mellitus), obesity, dyslipidemia, and steatohepatitis; cardiovascular diseases such as arteriosclerosis, myocardial infarction, cerebral infarction, and high blood pressure; cancers; renal disorders; osteodystrophy; cartilage tissue diseases; and autoimmune diseases. Hence, the compound of the present invention represented by General Formula (1) may be used as the active component of agents for preventing, improving, or treating these diseases.

The present invention provides use of the compound of General Formula (1) for production of a cytoprotectant against endoplasmic reticulum stress, the compound of General Formula (1) for use in a cytoprotection method against endoplasmic reticulum stress, and a cytoprotection method against endoplasmic reticulum stress by administering the compound of General Formula (1) in an effective amount for cytoprotection against endoplasmic reticulum stress to a patient having a disease caused by endoplasmic reticulum stress or a human subjected to endoplasmic reticulum stress.

The present invention also provides use of the compound of General Formula (1) for production of an agent for alleviating or reducing endoplasmic reticulum stress, the compound of General Formula (1) for use in a method of alleviating or reducing endoplasmic reticulum stress, and a method of alleviating or reducing endoplasmic reticulum stress by administering the compound of General Formula (1) in an effective amount for alleviating or reducing endoplasmic reticulum stress to a patient having a disease caused by endoplasmic reticulum stress or a human subjected to endoplasmic reticulum stress.

The present invention also provides use of the compound of General Formula (1) for production of a chemical chaperone and the compound of General Formula (1) for use as a chemical chaperone.

The present invention also provides use of the compound of General Formula (1) for production of an agent for preventing, improving, or treating a disease caused by endoplasmic reticulum stress, the compound of General Formula (1) for use in a method of preventing, improving, or treating a disease caused by endoplasmic reticulum stress, and a method of preventing, improving, or treating a disease caused by endoplasmic reticulum stress by administering the compound of General Formula (1) in an effective amount for preventing, improving, or treating a disease caused by endoplasmic reticulum stress to a patient having a disease caused by endoplasmic reticulum stress or a human exhibiting a sign of a disease caused by endoplasmic reticulum stress.

The cytoprotectant of the present invention against endoplasmic reticulum stress, the agent for alleviating or reducing endoplasmic reticulum stress, the chemical chaperone, and the agent for preventing, improving, or treating a disease caused by endoplasmic reticulum stress may be formulated as a pharmaceutical preparation in various dosage forms. Examples of the dosage form include oral preparations such as tablets, capsules, granules, powdered drugs, and syrups; and parenteral preparations such as injections (including intravenous injections, intramuscular injections, intradermal injections, subcutaneous injections, intramuscular injections, intrathecal injections, and intravenous infusions), enteral preparations via feeding tubes, suppositories, inhalants, and gargles. The pharmaceutical preparation may contain, in addition to the compound of General Formula (1), pharmaceutically acceptable carriers or excipients.

The dosage of the compound of General Formula (1) may vary depending on the type or severity of a disease or symptom, the dosage form, the administration route, or the like and may be appropriately determined by a person skilled in the art. For example, the daily dosage may be 0.00001 mg or more, 0.0001 mg or more, 0.001 mg or more, 0.01 mg or more, 0.1 mg or more, 1 mg or more, or 10 mg or more, and the daily dosage may be 10 g or less, 1000 mg or less, 100 mg or less, 10 mg or less, 1 mg or less, 0.1 mg or less, 0.01 mg or less, or 0.001 mg or less.

### EXAMPLES

Hereinafter, the present invention will be described in further detail, but the present invention is not limited to them.

### (1) Synthesis of benzothiazoimidazolyl compound

### IBT-30

A compound IBT-30 (compound 4) having the following structure was synthesized in the following manner.

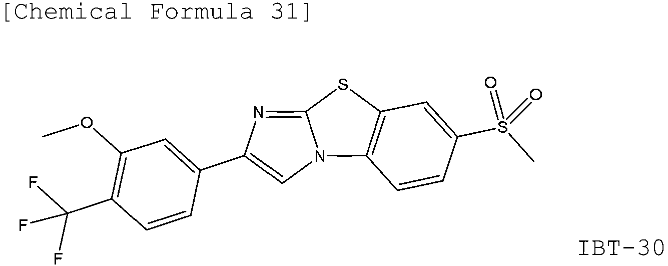

### 2-(3-Methoxy-4-(trifluoromethyl)phenyl)-7-(methylsulfonyl)benzo[d]imidazo[2,1-b]thiazole

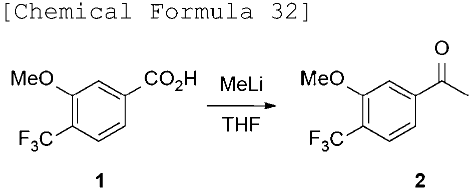

To a solution of a compound 1 (15.7 g, 71.1 mmol, COMBI-BLOCKS INC.) in tetrahydrofuran (160 ml) on ice, methyllithium (3.1M ethanol solution, 50 ml) was added dropwise through a dropping funnel. The mixture was stirred on ice for 2 hours, and the reaction was stopped with 1N-HCl. The reaction mixture was extracted with ethyl acetate, and the extract was washed with brine. Each aqueous layer was reextracted with ethyl acetate, and the extract was dried over magnesium sulfate. The desiccant was filtered off, and the solvent was removed under reduced pressure. The resulting residue was purified by high performance liquid chromatography (hexane/ethyl acetate) to give a compound 2 (13.8 g, yield: 91%).

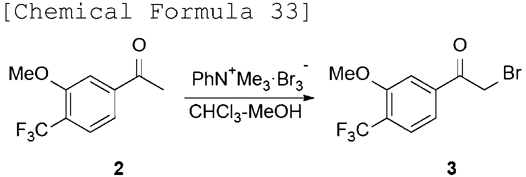

Under a nitrogen atmosphere, chloroform (80 ml) and methanol (30 ml) were added to the compound 2 (8.84 g, 40.5 mmol) at room temperature, and the mixture was stirred. PhN⁺Me₃·Br₃⁻ (15.2 g, 40.5 mmol, TCI) was further added at room temperature, and the mixture was stirred overnight. The solvent was removed under reduced pressure, and the resulting residue was purified by high performance liquid chromatography (hexane/ethyl acetate) to give a compound 3 (7.73 g, yield: 64%).

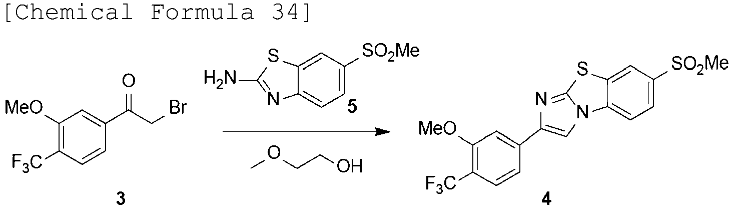

Under a nitrogen atmosphere, 6-methylsulfonyl-2-aminobenzothiazole (5.45 g, 23.6 mmol, Wako) and 2-methoxyethanol (55 ml) were added to the compound 3 (7.72 g, 20.6 mmol), and the mixture was heated and stirred on an oil bath at 150°C. The mixture was further stirred at 150°C for 4 hours and was allowed to cool. To the reaction mixture, 9% NH₃aq (150 ml) was added, and the mixture was stirred for a while. After the precipitate formed fine particles, the fine particles were separated by filtration. To the fine particles, a mixture of methanol/water (1:1, 50 ml) was added again, and the whole was stirred. The precipitate was separated by filtration and then was dried under reduced pressure. The resulting crude product was dissolved in tetrahydrofuran, and then dust or the like was filtered off. After concentration, methanol (20 ml) was added to prepare a slurry. After filtration, the solvent was removed under reduced pressure to give a compound 4 (IBT-30, 4.46 g, yield: 44%).

The NMR analysis result of the compound 4 (IBT-30) is shown below.
400 MHz
¹H-NMR (DMSO-D₆) □: 9.10 (1H, s), 8.74 (1H, d, J = 1.7 Hz), 8.21 (1H, d, J = 8.6 Hz), 8.15 (1H, dd, J = 8.6, 1.7 Hz), 7.72 (1H, s), 7.69 (1H, d, J = 8.1 Hz), 7.59 (1H, d, J = 8.1 Hz), 3.99 (3H, s), 3.31 (3H, s).

The same synthetic method as for the compound IBT-30 was performed except that the substituents on the benzene ring of the compound 1 and/or the substituents on the benzene ring of the compound 5 in the synthetic method of the compound IBT-30 described above were replaced with intended substituents, and compounds IBT-31 to 41, IBT-43 to 46, IBT-51 to 53, IBT-55 to 61, IBT-63, IBT-64, IBT-66 to 68, IBT-70, IBT-72 to 74, and IBT-76 were synthesized.

The chemical names, the structural formulae, and the NMR analysis results of these compounds are shown below.

### IBT-31

### 7-(methylsulfonyl)-2-(4-(trifluoromethyl)phenyl)benzo[d]imidazo[2,1-b]thiazole

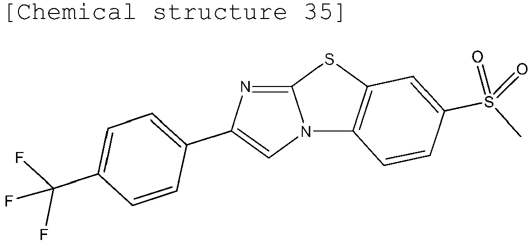

400MHz
1H-NMR (DMSO-D6) δ: 9.08 (1H, s), 8.74 (1H, d, J = 1.6 Hz), 8.24 (1H, d, J = 8.3 Hz), 8.14 (1H, dd, J = 8.3, 1.6 Hz), 8.09 (2H, d, J = 8.2 Hz), 7.82 (2H, d, J = 8.2 Hz), 3.31 (3H, s).

### IBT-32

### 2-(3,4-dichlorophenyl)-7-(methylsulfonyl)benzo[d]imidazo[2,1-b]thiazole

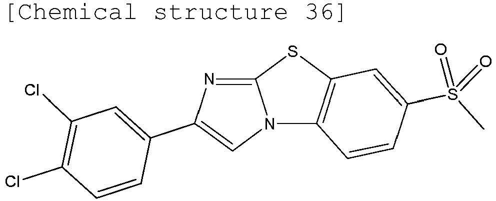

400MHz
1H-NMR (DMSO-D6) δ: 9.04 (1H, s), 8.74 (1H, dd, J = 1.7, 0.6 Hz), 8.17 (1H, dd, J = 8.4, 0.6 Hz), 8.14 (1H, dd, J = 8.4, 1.7 Hz), 8.09 (1H, d, J = 2.1 Hz), 7.85 (1H, dd, J = 8.3, 2.1 Hz), 7.73 (1H, d, J = 8.3 Hz), 3.30 (3H, s).

### IBT-33

### 2-(3-methoxyphenyl)-7-(methylsulfonyl)benzo[d]imidazo[2,1-b]thiazole

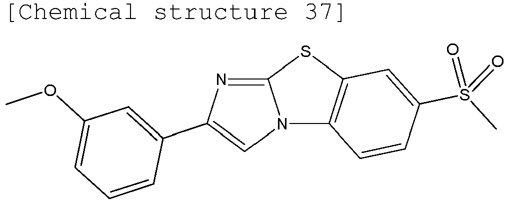

400MHz
1H-NMR (DMSO-D6) δ: 8.91 (1H, s), 8.72 (1H, d, J = 1.6 Hz), 8.20 (1H, d, J = 8.3 Hz), 8.13 (1H, dd, J = 8.3, 1.6 Hz), 7.47-7.45 (2H, m), 7.37 (1H, t, J = 8.0 Hz), 6.91-6.88 (1H, m), 3.30 (3H, s).

### IBT-34

### 2-(4-fluorophenyl)-7-(methylsulfonyl)benzo[d]imidazo[2,1-b]thiazole

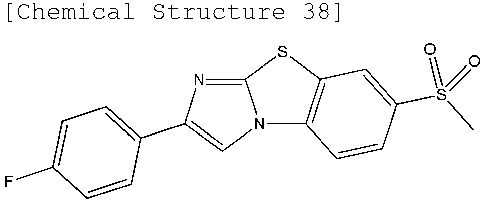

400MHz
1H-NMR (DMSO-D6) δ: 8.87 (1H, s), 8.72 (1H, d, J = 1.6 Hz), 8.20 (1H, d, J = 8.6 Hz), 8.12 (1H, dd, J = 8.6, 1.6 Hz), 7.92 (1H, d, J = 5.5 Hz), 7.90 (1H, d, J = 5.5 Hz), 7.31 (1H, d, J = 8.6 Hz), 7.28 (1H, d, J = 8.6 Hz), 3.30 (3H, s).

### IBT-35

### 3-(2-(4-chlorophenyl)benzo[d]imidazo[2,1-b]thiazol-7-yl)-N-methylpropanamide

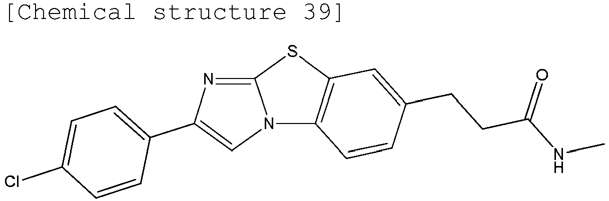

400MHz
1H-NMR (DMSO-D6) δ: 8.78 (1H, s), 7.88-7.85 (4H, m), 7.76 (1H, t, J = 4.3 Hz), 7.49 (2H, d, J = 8.6 Hz), 7.40 (1H, dd, J = 8.3, 1.6 Hz), 2.93 (2H, t, J = 7.7 Hz), 2.55 (3H, d, J = 4.3 Hz), 2.43 (2H, t, J = 7.7 Hz).

### IBT-36

### 2-(4-chloro-3-methoxyphenyl)-7-(methylsulfonyl)benzo[d]imidazo[2,1-b]thiazole

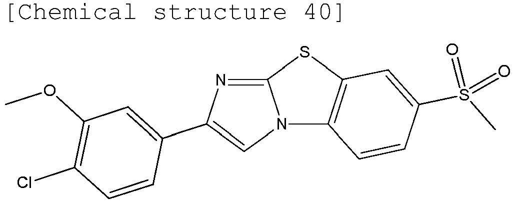

400MHz
1H-NMR (DMSO-D6) δ: 8.98 (1H, s), 8.73 (1H, d, J = 1.7 Hz), 8.19 (1H, d, J = 8.6 Hz), 8.14 (1H, dd, J = 8.6, 1.7 Hz), 7.63 (1H, d, J = 1.7 Hz), 7.50 (1H, d, J = 8.2 Hz), 7.46 (1H, dd, J = 8.2, 1.7 Hz), 3.96 (3H, s), 3.30 (3H, s).

### IBT-37

### N-methyl-3-(2-(4-(trifluoromethyl)phenyl)benzo[d]imidazo[2,1-b]thiazol-7-yl)propanamide

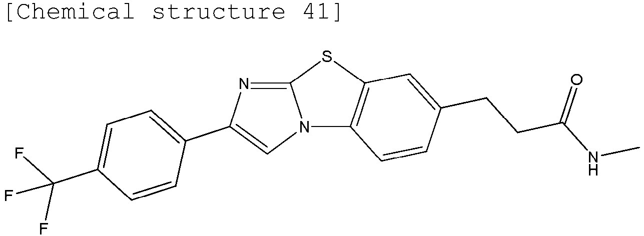

400MHz
1H-NMR (DMSO-D6) δ: 8.93 (1H, s), 8.07 (2H, d, J = 8.1 Hz), 7.90 (1H, d, J = 8.3 Hz), 7.87 (1H, d, J = 1.2 Hz), 7.80 (2H, d, J = 8.1 Hz), 7.76 (1H, q, J = 4.4 Hz), 7.41 (1H, dd, J = 8.3, 1.2 Hz), 2.94 (2H, t, J = 7.6 Hz), 2.56 (3H, d, J = 4.4 Hz), 2.43 (2H, t, J = 7.6 Hz).

### IBT-38

### N-methyl-4-((2-(4-(trifluoromethyl)phenyl)benzo[d]imidazo[2,1-b]thiazol-7-yl)sulfonyl)butanamide

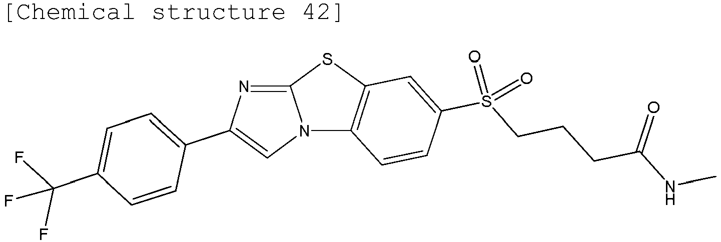

400MHz
1H-NMR (DMSO-D6) δ: 9.10 (1H, s), 8.71 (1H, dd, J = 1.8, 0.5 Hz), 8.24 (1H, d, J = 8.6 Hz), 8.11-8.08 (3H, m), 7.83 (2H, d, J = 8.1 Hz), 7.74 (1H, s), 3.40-3.36 (2H, m), 2.52 (3H, d, J = 4.6 Hz), 2.17 (2H, t, J = 7.4 Hz), 1.82-1.74 (2H, m).

### IBT-39

### N,N-diethyl-3-(2-(4-(trifluoromethyl)phenyl)benzo[d]imidazo[2,1-b]thiazol-7-yl)propanamide

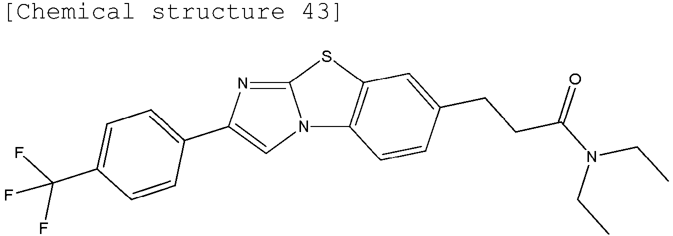

400MHz
1H-NMR (DMSO-D6) δ: 8.93 (1H, s), 8.07 (2H, d, J = 8.3 Hz), 7.91-7.89 (2H, m), 7.80 (2H, d, J = 8.3 Hz), 7.47 (1H, dd, J = 8.3, 1.4 Hz), 3.29-3.24 (4H, m), 2.95 (2H, t, J = 7.6 Hz), 2.66 (2H, t, J = 7.6 Hz), 1.05 (3H, t, J = 7.1 Hz), 1.00 (3H, t, J = 7.1 Hz).

### IBT-40

### 2-(4-(trifluoromethyl)phenyl)benzo[d]imidazo[2,1-b]thiazole

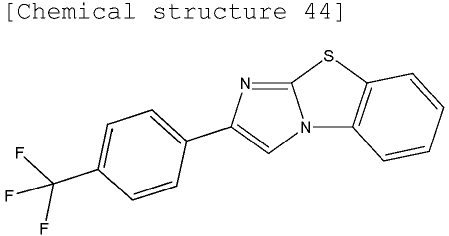

400MHz
1H-NMR (DMSO-D6) δ: 8.98 (1H, s), 8.08 (2H, d, J = 8.1 Hz), 8.07-8.05 (1H, m), 8.01 (1H, dt, J = 8.1, 0.6 Hz), 7.80 (2H, d, J = 8.1 Hz), 7.62-7.57 (1H, m), 7.48-7.44 (1H, m).

### IBT-41

### 7-(methylsulfonyl)-2-(4-(prop-2-yn-1-yloxy)phenyl)benzo[d]imidazo[2,1-b]thiazole 2,2,2-trifluoroacetate

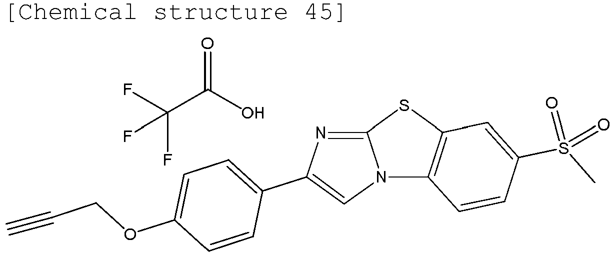

400MHz
1H-NMR (DMSO-D6) δ: 8.77 (1H, s), 8.70 (1H, d, J = 1.7 Hz), 8.20 (1H, d, J = 8.6 Hz), 8.11 (1H, dd, J = 8.6, 1.7 Hz), 7.82 (2H, d, J = 9.0 Hz), 7.08 (2H, d, J = 9.0 Hz), 4.84 (2H, d, J = 2.3 Hz), 3.59 (2H, t, J = 2.3 Hz), 3.29 (3H, s).

### IBT-43

### 2-(4-methoxyphenyl)-7-(methylsulfonyl)benzo[d]imidazo[2,1-b] thiazole

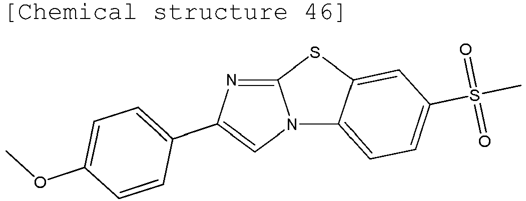

400MHz
1H-NMR (DMSO-D6) δ: 8.76 (1H, s), 8.70 (1H, d, J = 1.6 Hz), 8.19 (1H, d, J = 8.6 Hz), 8.11 (1H, dd, J = 8.6, 1.6 Hz), 7.81 (2H, d, J = 8.8 Hz), 7.03 (2H, d, J = 8.8 Hz), 3.80 (3H, s), 3.30 (3H, s).

### IBT-44

### 2-(2,4-dichlorophenyl)-7-(methylsulfonyl)benzo[d]imidazo[2,1-b]thiazole

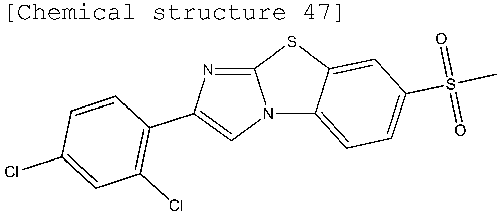

400MHz
1H-NMR (DMSO-D6) δ: 9.12 (1H, s), 8.73 (1H, d, J = 1.8 Hz), 8.48 (1H, d, J = 8.4 Hz), 8.23 (1H, d, J = 8.8 Hz), 8.13 (1H, dd, J = 8.4, 1.8 Hz), 7.74 (1H, d, J = 2.3 Hz), 7.55 (1H, dd, J = 8.8, 2.3 Hz), 3.31 (3H, s).

### IBT-45

### ethyl 2-(4-chlorophenyl)benzo[d]imidazo[2,1-b]thiazole-7-carboxylate

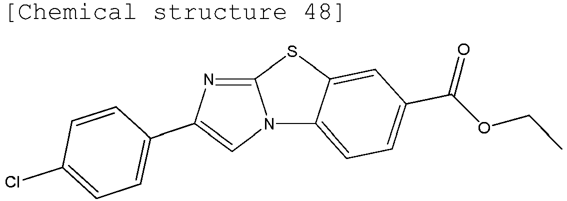

400MHz
1H-NMR (DMSO-D6) δ: 8.89 (1H, s), 8.70 (1H, dd, J = 1.6, 0.5 Hz), 8.15 (1H, dd, J = 8.6, 1.6 Hz), 8.06 (1H, dd, J = 8.6, 0.5 Hz), 7.89 (2H, d, J = 8.8 Hz), 7.51 (2H, d, J = 8.8 Hz), 4.37 (2H, d, J = 7.2 Hz), 1.36 (3H, t, J = 7.2 Hz).

### IBT-46

### 2-(4-chlorophenyl)-7-ethoxybenzo[d]imidazo[2,1-b]thiazole

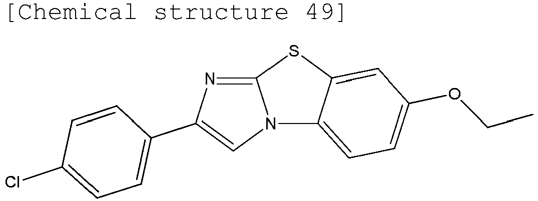

400MHz
1H-NMR (DMSO-D6) δ: 8.74 (1H, s), 7.86 (1H, d, J = 8.6 Hz), 7.85 (2H, d, J = 8.6 Hz), 7.66 (1H, d, J = 2.5 Hz), 7.49 (2H, d, J = 8.6 Hz), 7.14 (1H, dd, J = 8.6, 2.5 Hz), 4.10 (2H, d, J = 6.9 Hz), 1.37 (3H, t, J = 6.9 Hz).

### IBT-51

### 2-(4-chlorophenyl)-5-methoxybenzo[d]imidazo[2,1-b]thiazole

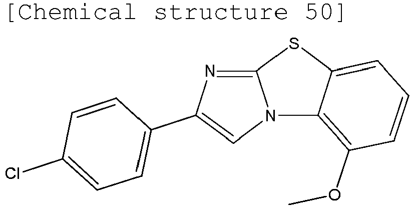

400MHz
1H-NMR (DMSO-D6) δ: 8.63 (1H, s), 7.96 (2H, d, J = 8.6 Hz), 7.59 (1H, dd, J = 8.1, 0.7 Hz), 7.47 (2H, d, J = 8.6 Hz), 7.39 (1H, t, J = 8.1 Hz), 7.22 (1H, dd, J = 8.1, 0.7 Hz), 4.08 (3H, s).

### IBT-52

### N-methyl-4-((2-(4-(trifluoromethyl)phenyl)benzo[d]imidazo[2,1-b]thiazole)-7-sulfonamido)butanamide

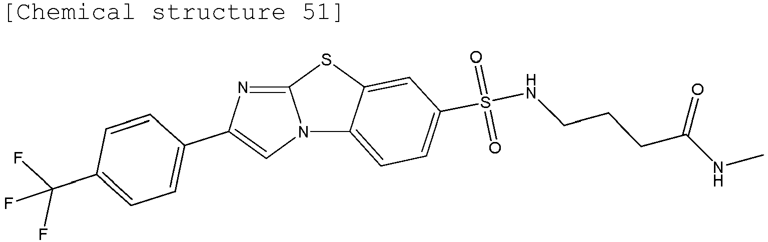

400MHz
1H-NMR (DMSO-D6) δ: 9.05 (1H, s), 8.57 (1H, d, J = 1.8 Hz), 8.18 (1H, d, J = 8.3 Hz), 8.09 (2H, d, J = 8.1 Hz), 7.97 (1H, dd, J = 8.3, 1.8 Hz), 7.82 (2H, d, J = 8.1 Hz), 7.74 (1H, t, J = 5.5 Hz), 7.68 (1H, br s), 2.81-2.77 (2H, m), 2.52 (3H, s), 2.05 (2H, t, J = 7.4 Hz), 1.64-1.57 (2H, m).

### IBT-53

### tert-butyl (2-(2-(3-(2-(4-(trifluoromethyl)phenyl)benzo[d]imidazo[2,1-b]thiazol-7-yl)propanamido)ethoxy)ethyl)carbamate

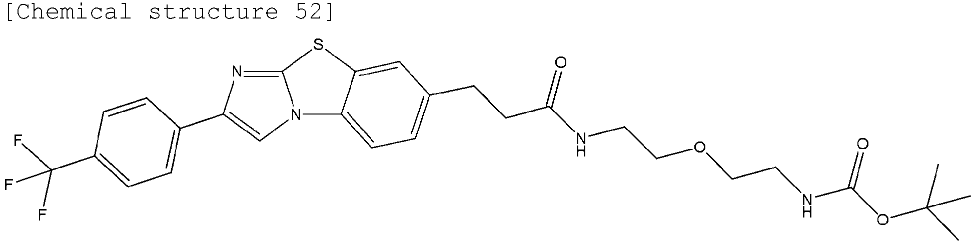

400MHz
1H-NMR (DMSO-D6) δ: 8.92 (1H, s), 8.06 (2H, d, J = 8.1 Hz), 7.90 (1H, d, J = 8.3 Hz), 7.90 (1H, s), 7.86 (1H, d, J = 1.4 Hz), 7.79 (2H, d, J = 8.1 Hz), 7.42 (1H , dd, J = 8.3, 1.4 Hz), 6.75 (1H , t, J = 5.3 Hz), 3.36-3.33 (4H, m), 3.18 (2H, d, J = 5.6 Hz), 3.04 (2H, d, J = 5.8 Hz), 2.95 (2H, t, J = 7.5 Hz), 2.46 (2H, t, J = 7.6 Hz), 1.35 (9H, s).

### IBT-55

### N,N-diethyl-4-((2-(4-(trifluoromethyl)phenyl)benzo[d]imidazo[2,1-b]thiazol-7-yl)sulfonyl)butanamide

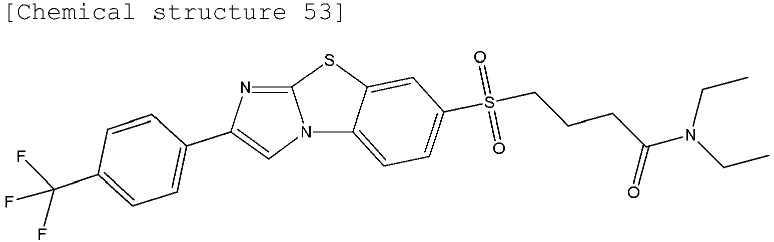

400MHz
1H-NMR (DMSO-D6) δ: 9.09 (1H, s), 8.72 (1H, d, J = 1.6 Hz), 8.25 (1H, d, J = 8.6 Hz), 8.09 (2H, d, J = 8.3 Hz), 8.09 (1H, dd, J = 8.6, 1.6 Hz), 7.83 (2H, d, J = 8.3 Hz), 3.44-3.40 (2H, m), 3.21 (4H, q, J = 7.2 Hz), 2.40 (2H, t, J = 7.1 Hz), 1.83-1.75 (2H, m), 1.04 (3H, t, J = 7.2 Hz), 0.96 (3H, t, J = 7.2 Hz).

### IBT-56

### 2-(4-chloro-3-(prop-2-yn-1-yloxy)phenyl)-7-(methylsulfonyl)benzo[d]imidazo[2,1-b]thiazole

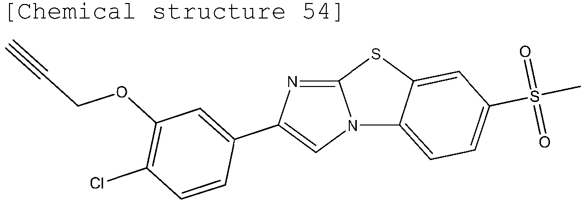

400MHz
1H-NMR (DMSO-D6) δ: 8.95 (1H, s), 8.73 (1H, dd, J = 1.8, 0.5 Hz), 8.21 (1H, d, J = 8.5 Hz), 8.13 (1H, dd, J = 8.5, 1.8 Hz), 7.73 (1H, d, J = 1.6 Hz), 7.53 (1H, d, J = 8.2 Hz), 7.50 (1H, dd, J = 8.2, 1.6 Hz), 5.02 (2H, d, J = 2.3 Hz), 3.67 (1H, t, J = 2.3 Hz), 3.30 (3H, s).

### IBT-57

### N-(prop-2-yn-1-yl)-3-(2-(4-(trifluoromethyl)phenyl)benzo[d]imidazo[2,1-b]thiazol-7-yl)propenamide

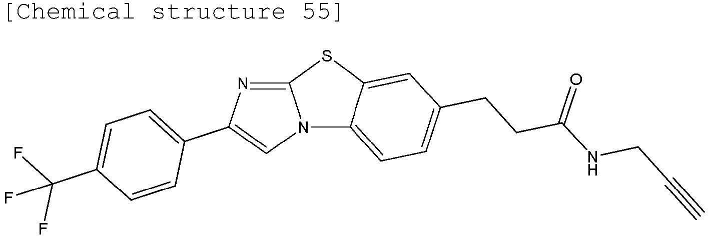

400MHz
1H-NMR (DMSO-D6) δ: 8.93 (1H, s), 8.31 (1H, t, J = 5.5 Hz), 8.07 (2H, d, J = 8.1 Hz), 7.90 (1H, d, J = 8.3 Hz), 7.86 (1H, d, J = 1.4 Hz), 7.80 (2H, d, J = 8.1 Hz), 7.42 (1H, dd, J = 8.3, 1.4 Hz), 3.85 (2H, dd, J = 5.5, 2.5 Hz), 3.09 (1H, t, J = 2.5 Hz), 2.95 (2H, t, J = 7.4 Hz), 2.46 (2H, t, J = 7.4 Hz).

### IBT-58

### tert-butyl (3-(2-chloro-5-(7-(methylsulfonyl)benzo[d]imidazo[2,1-b]thiazol-2-yl)phenoxy)propyl)carbamate

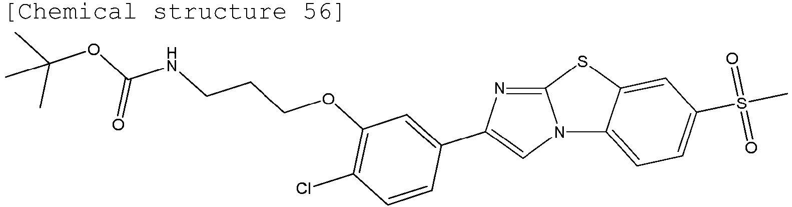

400MHz
1H-NMR (DMSO-D6) δ: 8.96 (1H, s), 8.73 (1H, d, J = 1.4 Hz), 8.18 (1H, d, J = 8.3 Hz), 8.14 (1H, dd, J = 8.3, 1.4 Hz), 7.62 (1H, d, J = 1.6 Hz), 7.50 (1H, d, J = 8.1 Hz), 7.45 (1H, dd, J = 8.1, 1.6 Hz), 6.93 (1H, t, J = 5.4 Hz), 4.17 (2H, t, J = 6.2 Hz), 3.30 (3H, s), 3.17-3.14 (2H, m), 1.95-1.89 (2H, m), 1.38 (9H, s).

### IBT-59

### (2-(4-chlorophenyl)benzo[d]imidazo[2,1-b]thiazol-7-yl)methanol

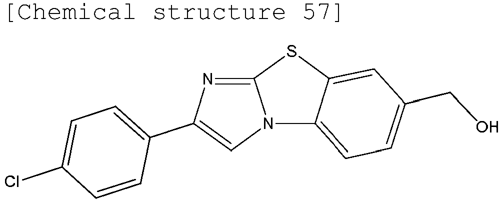

400MHz
1H-NMR (DMSO-D6) δ: 8.80 (1H, t, J = 4.3 Hz), 7.97-7.96 (1H, m), 7.92 (1H, d, J = 8.3 Hz), 7.88 (2H, d, J = 8.9 Hz), 7.52-7.48 (1H, m), 7.50 (2H, d, J = 8.9 Hz), 5.39 (1H, t, J = 5.7 Hz), 4.61 (2H, d, J = 5.6 Hz).

### IBT-60

### 2-(4-chlorophenyl)-7-((prop-2-yn-1-yloxy)methyl)benzo[d]imidazo[2,1-b]thiazole 2,2,2-trifluoroacetate

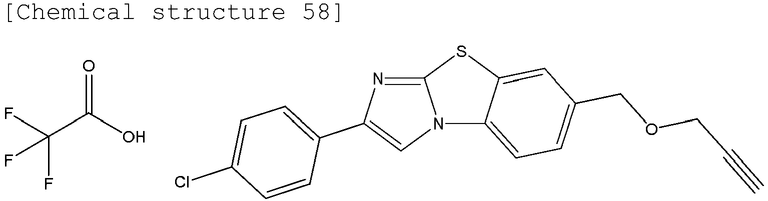

400MHz
1H-NMR (DMSO-D6) δ: 8.82 (1H, t, J = 4.3 Hz), 8.01 (1H, d, J = 1.6 Hz), 7.96 (1H, d, J = 8.2 Hz), 7.88 (2H, d, J = 8.8 Hz), 7.53 (1H, dd, J = 8.2, 1.6 Hz), 7.50 (2H, d, J = 8.8 Hz), 4.64 (2H, s), 4.24 (2H, d, J = 2.3 Hz), 3.52 (1H, t, J = 2.3 Hz).

### IBT-61

### N-(2-(3-(but-3-yn-1-yl)-3H-diazirin-3-yl)ethyl)-3-(2-(4-(trifluoromethyl)phenyl)benzo[d]imidazo[2,1-b]thiazol-7-yl)propanamide

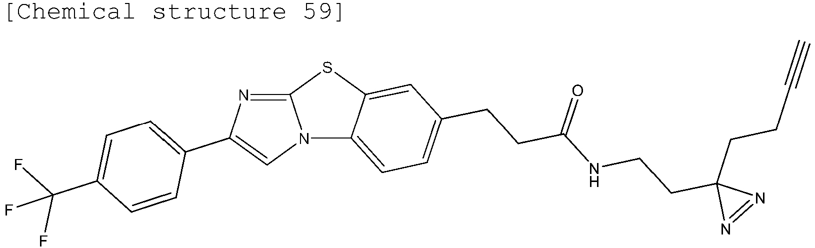

400MHz
1H-NMR (DMSO-D6) δ: 8.93 (1H, s), 8.06 (2H, d, J = 8.2 Hz), 7.90 (1H, d, J = 8.1 Hz), 7.86 (1H, d, J = 1.6 Hz), 7.86 (1H, t, J = 5.3 Hz), 7.79 (2H, d, J = 8.2 Hz), 7.42 (1H, dd, J = 8.1, 1.6 Hz), 2.97-2.88 (4H, m), 2.81 (1H, t, J = 2.7 Hz), 2.44 (2H, t, J = 7.5 Hz), 1.95 (2H, td, J = 7.3, 2.7 Hz), 1.54 (2H, t, J = 7.3 Hz), 1.48 (2H, t, J = 7.2 Hz).

### IBT-63

### 2-(3-(2-(3-(but-3-yn-1-yl)-3H-diazirin-3-yl)ethoxy)-4-chlorophenyl)-7-(methylsulfonyl)benzo[d]imidazo[2,1-b]thiazole 2,2,2-trifluoroacetate

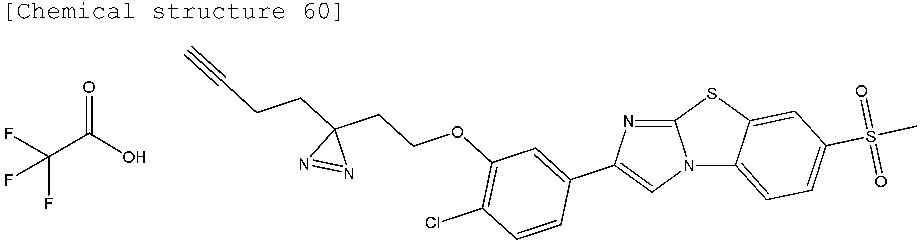

400MHz
1H-NMR (DMSO-D6) δ: 8.97 (1H, s), 8.73 (1H, d, J = 1.7 Hz), 8.18 (1H, d, J = 8.4 Hz), 8.14 (1H, dd, J = 8.4, 1.7 Hz), 7.60 (1H, d, J = 1.7 Hz), 7.52 (1H, d, J = 8.2 Hz), 7.48 (1H, dd, J = 8.2, 1.7 Hz), 4.05 (2H, t, J = 5.9 Hz), 3.30 (3H, s), 2.84 (1H, t, J = 2.7 Hz), 2.08 (2H, td, J = 7.5, 2.7 Hz), 1.93 (2H, t, J = 5.9 Hz), 1.75 (2H, t, J = 7.5 Hz).

### IBT-64

### 2-(4-ethoxyphenyl)-7-(methylsulfonyl)benzo[d]imidazo[2,1-b]thiazole

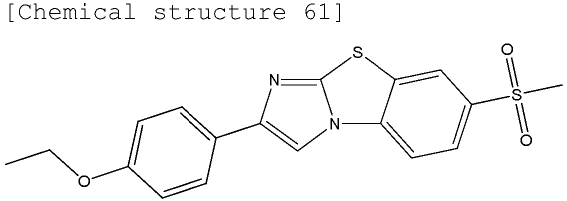

400MHz
1H-NMR (DMSO-D6) δ: 8.75 (1H, s), 8.70 (1H, d, J = 1.8 Hz), 8.19 (1H, d, J = 8.6 Hz), 8.11 (1H, dd, J = 8.6, 1.8 Hz), 7.79 (2H, d, J = 8.8 Hz), 7.01 (2H, d, J = 8.8 Hz), 4.07 (2H, d, J = 7.0 Hz), 3.29 (3H, s), 1.35 (3H, t, J = 7.0 Hz).

### IBT-66

### tert-butyl (3-(4-(7-(methylsulfonyl)benzo[d]imidazo[2,1-b]thiazol-2-yl)phenoxy)propyl)carbamate

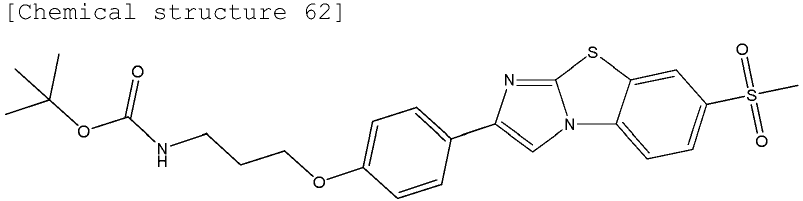

400MHz
1H-NMR (DMSO-D6) δ: 8.76 (1H, s), 8.70 (1H, d, J = 1.8 Hz), 8.19 (1H, d, J = 8.3 Hz), 8.11 (1H, dd, J = 8.3, 1.8 Hz), 7.80 (2H, d, J = 8.9 Hz), 7.01 (2H, d, J = 8.9 Hz), 6.90 (1H, t, J = 4.9 Hz), 4.02 (2H, t, J = 6.2 Hz), 3.29 (3H, s), 3.12-3.08 (2H, m), 1.88-1.82 (2H, m), 1.38 (9H, s).

### IBT-67

### 7-(methylsulfonyl)-2-(4-propylphenyl)benzo[d]imidazo[2,1-b]thiazole

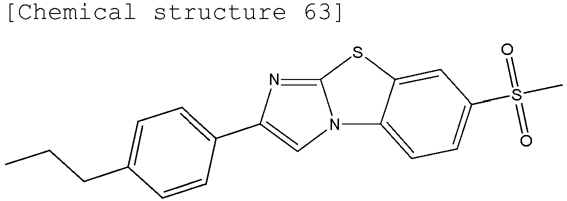

400MHz
1H-NMR (DMSO-D6) δ: 8.83 (1H, s), 8.71 (1H, dd, J = 1.8, 0.5 Hz), 8.21 (1H, dd, J = 8.6, 0.5 Hz), 8.12 (1H, dd, J = 8.6, 1.8 Hz), 7.79 (2H, d, J = 8.3 Hz), 7.27 (2H, d, J = 8.3 Hz), 3.30 (3H, s), 2.59 (2H, t, J = 7.5 Hz), 1.66-1.59 (2H, m), 0.92 (3H, t, J = 7.3 Hz).

### IBT-68

### 7-methyl-2-(4-(trifluoromethyl)phenyl)benzo[d]imidazo[2,1-b]thiazole

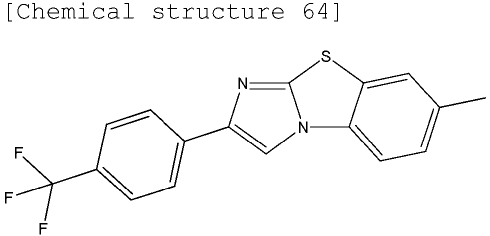

400MHz
1H-NMR (DMSO-D6) δ: 8.92 (1H, s), 8.06 (2H, d, J = 8.1 Hz), 7.88 (1H, d, J = 8.1 Hz), 7.85 (1H, s), 7.79 (2H, d, J = 8.1 Hz), 7.40 (1H, d, J = 8.1 Hz), 2.44 (3H, s).

### IBT-70

### 2-(3-(2-(3-(but-3-yn-1-yl)-3H-diazirin-3-yl)ethoxy)phenyl)-7-(methylsulfonyl)benzo[d]imidazo[2,1-b]thiazole 2,2,2-trifluoroacetate

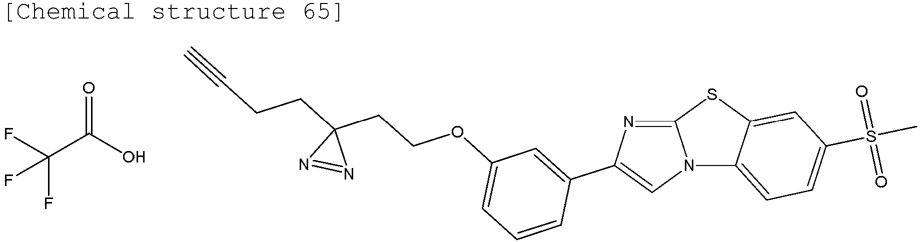

400MHz
1H-NMR (DMSO-D6) δ: 8.92 (1H, s), 8.72 (1H, d, J = 1.4 Hz), 8.20 (1H, d, J = 8.4 Hz), 8.13 (1H, dd, J = 8.4, 1.4 Hz), 7.49-7.44 (2H, m), 7.36 (1H, t, J = 8.0 Hz), 6.90-6.87 (1H, m), 3.90 (2H, t, J = 6.1 Hz), 3.30 (3H, s), 2.84 (1H, t, J = 2.7 Hz), 2.07 (2H, td, J = 7.4, 2.7 Hz), 1.92 (2H, t, J = 6.1 Hz), 1.70 (2H, t, J = 7.4 Hz).

### IBT-72

### 8-bromo-2-(4-(trifluoromethyl)phenyl)benzo[d]imidazo[2,1-b]thiazole

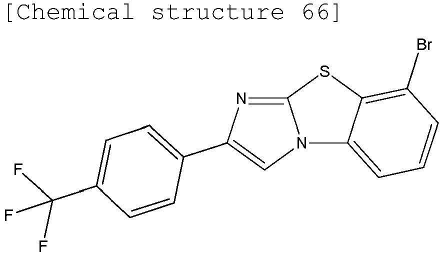

400MHz
1H-NMR (DMSO-D6) δ: 8.98 (1H, s), 8.07 (2H, d, J = 8.1 Hz), 8.04 (1H, dd, J = 8.2, 0.9 Hz), 7.80 (2H, d, J = 8.1 Hz), 7.70 (1H, dd, J = 8.2, 0.9 Hz), 7.58 (1H, t, J = 8.2 Hz).

### IBT-73

### N-(2-(3-(but-3-yn-1-yl)-3H-diazirin-3-yl)ethyl)-3-(2-(4-(trifluoromethyl)phenyl)benzo[d]imidazo[2,1-b]thiazol-8-yl)propanamide 2,2,2-trifluoroacetate

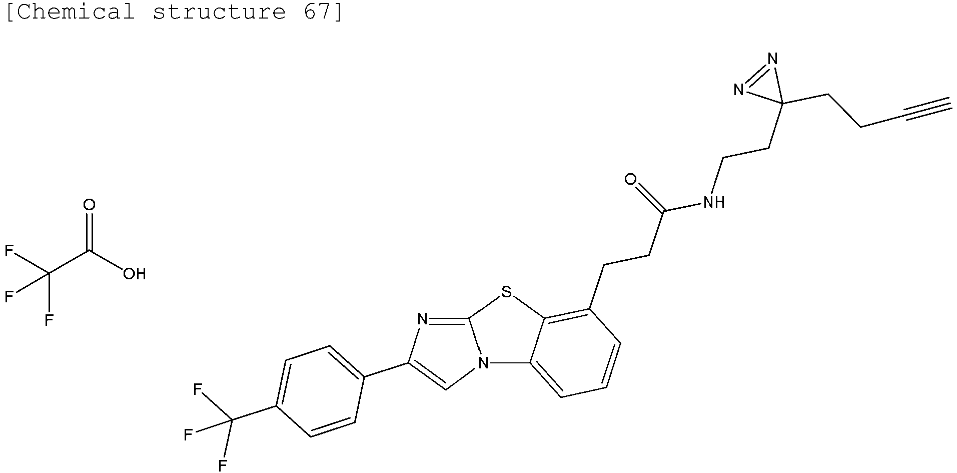

400MHz
1H-NMR (DMSO-D6) δ: 8.97 (1H, s), 8.08 (2H, d, J = 8.6 Hz), 7.93 (1H, t, J = 5.4 Hz), 7.87 (1H, d, J = 7.8 Hz), 7.80 (2H, d, J = 8.6 Hz), 7.54 (1H, t, J = 7.8 Hz), 7.31 (1H, d, J = 7.8 Hz), 3.01 (2H, t, J = 7.5 Hz), 2.92 (2H, dt, J = 5.4, 7.4 Hz), 2.81 (1H, t, J = 2.4 Hz), 2.54 (3H, t, J = 7.5 Hz), 1.96 (2H, td, J = 7.1, 2.4 Hz), 1.55 (2H, t, J = 7.4 Hz), 1.49 (2H, t, J = 7.1 Hz).

### IBT-74

### tert-butyl (2-(2-(3-(2-(4-(trifluoromethyl)phenyl)benzo[d]imidazo[2,1-b]thiazol-8-yl)propanamido)ethoxy)ethyl)carbamate

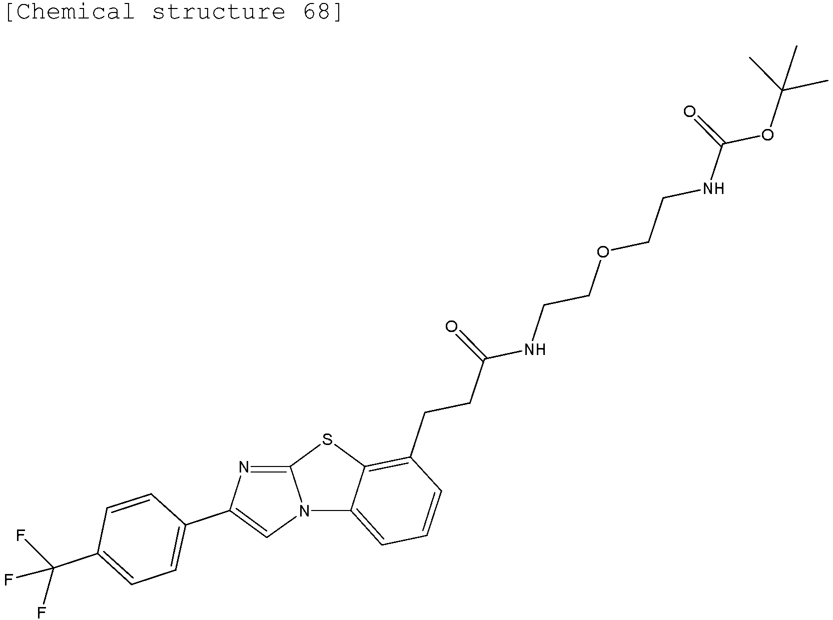

400MHz
1H-NMR (DMSO-D6) δ: 8.98 (1H, s), 8.08 (2H, d, J = 8.1 Hz), 7.98 (1H, t, J = 5.5 Hz), 7.87 (1H, dd, J = 7.7, 1.0 Hz), 7.80 (2H, d, J = 8.1 Hz), 7.54 (1H, t, J = 7.7 Hz), 7.31 (1H, d, J = 7.7 Hz), 6.75 (1H, t, J = 6.0 Hz), 3.36 (2H, t, J = 5.8 Hz), 3.35 (2H, t, J = 6.0 Hz), 3.19 (2H, dt, J = 5.5, 5.8 Hz), 3.05 (2H, dt, J = 6.0, 6.0 Hz), 3.01 (2H, t, J = 7.5 Hz), 2.56 (2H, t, J = 7.5 Hz), 1.36 (9H, s).

### IBT-76

### tert-butyl (2-(2-(2-(3-(7-(methylsulfonyl)benzo[d]imidazo[2,1-b]thiazol-2-yl)phenoxy)ethoxy)ethoxy)ethyl)carbamate

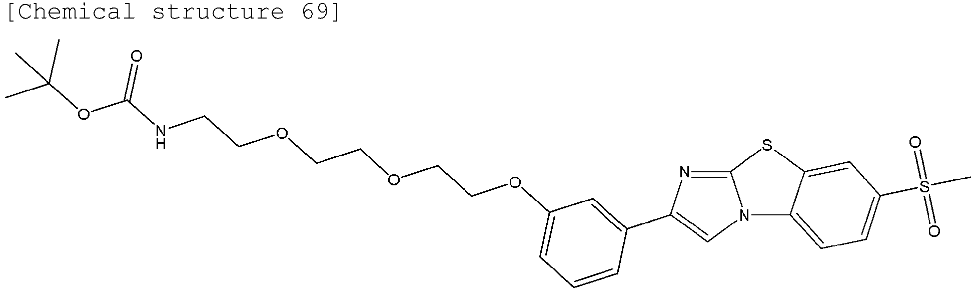

400MHz
1H-NMR (DMSO-D6) δ: 8.91 (1H, s), 8.72 (1H, dd, J = 1.8, 0.5 Hz), 8.18 (1H, dd, J = 8.1, 0.5 Hz), 8.13 (1H, dd, J = 8.1, 1.7 Hz), 7.48-7.46 (2H, m), 7.36 (1H, t, J = 8.1 Hz), 6.91-6.88 (1H, m), 6.75 (1H, t, J = 6.2 Hz), 4.18-4.15 (2H, m), 3.80-3.78 (2H, m), 3.63-3.60 (2H, m), 3.56-3.53 (2H, m), 3.40 (2H, t, J = 6.1 Hz), 3.29 (3H, s), 3.07 (2H, q, J = 6.1 Hz), 1.36 (9H, s).

### (2) Evaluation of activity

Tunicamycin (Tm) used in the following EXAMPLES was prepared as follows: a solution of Tm (FUJIFILM Wako Pure Chemical Corporation; No. 208-08243) at 2 µg/µL in DMSO was prepared and was further diluted 2000-fold with a DMEM solution containing 10% fetal bovine serum (FBS) to prepare a 1 ng/µL Tm solution. The test compounds (IBT-30 to 41, 43 to 46, 51 to 53, 55 to 61, 63 to 64, 66 to 68, 70 to 74, and 76) were used as a 2 mM DMSO solution. The control compound IBT-21 was used as a DMSO solution.

### Example 1 (Suppressive effect on endoplasmic reticulum stress response)

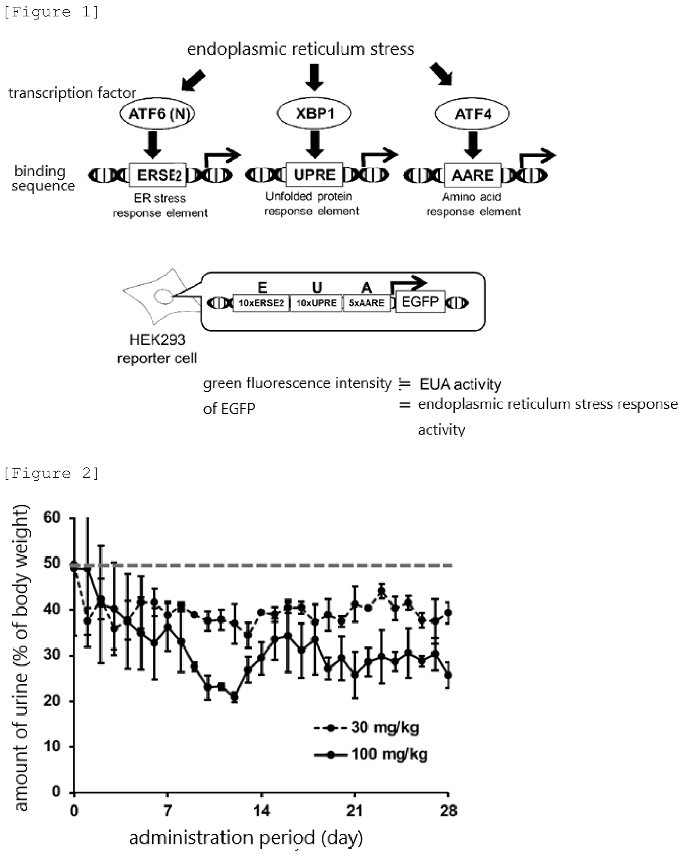

As shown in Fig. 1, based on a third-generation lentiviral vector, a vector comprising a sequence (10 × ERSE2 - 10 × UPRE - 5 × AARE - EGFP) exhibiting EGFP downstream of the tandem repeat of ER stress response element (ERSE2) targeted by ATF6, unfolded protein response element (UPRE) targeted by XBP1, and amino acid response element (AARE) targeted by ATF4 was created and was transfected into 293A cells to give reporter cells.
ERSE2: GGCGCCGATTGGGCCACGTTGGGAGAGTGCCT (SEQ ID NO: 1)
UPRE: CTCGAGACAGGTGCTGACGTGGCATTC (SEQ ID NO: 2)
AARE: AACATTGCATCATCCCCGC (SEQ ID NO: 3)

A solution of the compound synthesized in "(1) Synthesis of benzothiazoimidazolyl compound" was appropriately diluted with a DMEM solution containing 10% fetal bovine serum (FBS), and the diluted solution was added to a 384-well assay plate at 10 µL/well. Next, 30 µL of a culture medium containing the reporter cells and 10 µL of the Tm solution were mixed, and the mixture was added to the 384-well assay plate at 40 µL/well such that the cell density was 18 × 10³ cells/well, and the whole was incubated at 37°C overnight.

A multi-plate reader Citation 3 (Bio-Tek) was used to determine the EGFP fluorescence intensity (excitation wavelength: 485 nm, fluorescence wavelength: 528 nm) when the test compound was added. A smaller fluorescence intensity indicates more suppression of the increase in activity of the endoplasmic reticulum stress marker induced by Tm.

A "control" containing the Tm solution and, in place of the test compound solution, an equal volume of DMSO was subjected to the measurement of fluorescence intensity in a similar manner. In addition, a "background" containing a solution prepared in the same manner as for the Tm solution except that no Tm was added and containing, in place of the test compound solution, an equal volume of DMSO was subjected to the measurement of fluorescence intensity in a similar manner.

The fluorescence intensity was determined while the concentration of the test compound was varied, and the 50% effective concentration (IC₅₀) of suppressing the endoplasmic reticulum stress response induced by Tm was calculated. A test compound having an IC₅₀ of 0.01 µm or less was evaluated as rank A; a test compound having an IC₅₀ of more than 0.01 µM and not more than 0.1 µm was evaluated as rank B; a test compound having an IC₅₀ of more than 0.1 µM and not more than 1 µm was evaluated as rank C; and a test compound having an IC₅₀ of more than 1 µM was evaluated as rank D.

The results were shown in Table 1.

**[Table 1]**

| Compound | Suppression rank of endoplasmic reticulum stress response | Compound | Suppression rank of endoplasmic reticulum stress response | Compound | Suppression rank of endoplasmic reticulum stress response |
|---|---|---|---|---|---|
| IBT-30 | A | IBT-44 | C | IBT-63 | C |
| IBT-31 | A | IBT-45 | C | IBT-64 | C |
| IBT-32 | B | IBT-46 | C | IBT-66 | C |
| IBT-33 | B | IBT-51 | D | IBT-67 | C |
| IBT-34 | B | IBT-52 | C | IBT-68 | B |
| IBT-35 | B | IBT-53 | D | IBT-70 | C |
| IBT-36 | A | IBT-55 | C | IBT-72 | C |
| IBT-37 | B | IBT-56 | C | IBT-73 | D |
| IBT-38 | B | IBT-57 | D | IBT-74 | D |
| IBT-39 | B | IBT-58 | D | IBT-76 | D |
| IBT-40 | B | IBT-59 | C | | |
| IBT-41 | B | IBT-60 | C | IBT-21 | C |
| IBT-43 | C | IBT-61 | D | | |

The compound of the present invention strongly suppressed the endoplasmic reticulum stress response induced by Tm.

### Example 2 (Cytoprotection effect under endoplasmic reticulum stress conditions)

As the compounds, compounds IBT-30 to 41, IBT-43 to 46, IBT-51 to 53, IBT-55 to 61, IBT-63, IBT-64, IBT-66 to 68, IBT-70, IBT-72 to 74, and IBT-76 synthesized as above were used.

The reporter cells same as those used in Example 1 were placed in each well of a 96-well assay plate and were treated with Tm at a final concentration of 0.2 µg/mL and the test compound. The final concentration of each compound in the wells of the assay plate was varied to 20 µM, 10 µM, 5 µM, 2.5 µM, 1.25 µM, 625 nM, 313 nM, or 156 nM, and the compound concentration inhibiting Tm-induced cell death by 50% (IC₅₀) was calculated. The results were shown in Table 2.

**[Table 2]**

| Compound | IC₅₀ (µM) | Compound | IC₅₀ (µM) | Compound | IC₅₀ (µM) |
|---|---|---|---|---|---|
| IBT-30 | 0.004 | IBT-44 | 0.45 | IBT-63 | 0.35 |
| IBT-31 | 0.01 | IBT-45 | 0.35 | IBT-64 | 0.67 |
| IBT-32 | 0.02 | IBT-46 | 0.56 | IBT-66 | 0.83 |
| IBT-33 | 0.07 | IBT-51 | 7.72 | IBT-67 | 0.37 |
| IBT-34 | 0.08 | IBT-52 | 0.42 | IBT-68 | 0.1 |
| IBT-35 | 0.04 | IBT-53 | 4.47 | IBT-70 | 0.43 |
| IBT-36 | 0.01 | IBT-55 | 0.66 | IBT-72 | 0.63 |
| IBT-37 | 0.03 | IBT-56 | 0.56 | IBT-73 | 8.4 |
| IBT-38 | 0.09 | IBT-57 | 3.8 | IBT-74 | 3.7 |
| IBT-39 | 0.08 | IBT-58 | 3.2 | IBT-76 | 4.9 |
| IBT-40 | 0.1 | IBT-59 | 0.41 | IBT-21 | 0.61 |
| IBT-41 | 0.09 | IBT-60 | 0.9 | 4-PBA | 4000 |
| IBT-43 | 0.17 | IBT-61 | 4.16 | TUDCA | 20 |

Compounds of the present invention indicated by General Formula (1) effectively protected cells from cell death by the endoplasmic reticulum stress given by Tm.

### Example 3 (Effect of improving familial neurogenic diabetes insipidus)

### Suppression of urine output increases

Familial neurogenic diabetes insipidus model mice created by Arima et al. at Nagoya University and carrying a knock-in mutation (Cys98stop) in the neurophysin II gene that causes familial neurogenic diabetes insipidus (FNDI) in humans (Am J Physiol Regul Integr Comp Physiol 2009 296:R1641-9) were divided into two groups. To each group, an IBT-30-containing preparation was forcibly administered orally such that the dosage of IBT-30 was 30 mg/kg/day or 100 mg/kg/day (n=6). The IBT-30-containing preparation was prepared as a suspension of IBT-30 at a final concentration of 0.5% by weight in carboxymethyl cellulose. The oral administration was performed once daily via a feeding tube for 28 days. The urine output was measured daily throughout the 28 days when IBT-30 was administered.

The results are shown in Fig. 2. From the day after starting the administration of IBT-30, the urine output decreases in a dose-dependent manner with IBT-30.

### Decrease in the number of mutant protein aggregates in the brain tissue

To FNDI model mice, the above IBT-30-containing preparation was forcibly administered orally once daily for 28 days such that the dosage of IBT-30 was 100 mg/kg/day (n=6). In a similar manner, a vehicle preparation free from IBT-30 was forcibly administered orally (n=6). A frozen section of the suprachiasmatic nucleus from each mouse was used to identify vasopressin neurons stained with rabbit anti-mutant NPII antibody (1:1000), and the number of inclusion bodies having a dimension of 4.5 µm or more (mutant protein aggregates) of the vasopressin neurons was counted under an optical microscope.

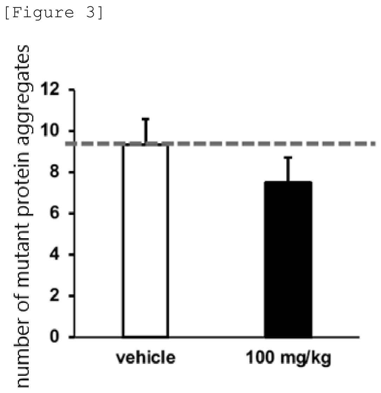

The results are shown in Fig. 3. Administration of IBT-30 significantly reduced the number of inclusion bodies (mutant protein aggregates). The decrease in the number of aggregates is thought to result from the inhibition of aggregate formation or the promotion of folded protein degradation. IBT-30 assisted in the molecular chaperone function that performs such inhibition or promotion in vivo, and this reveals that IBT-30 functioned as a chemical chaperone.

It has been ascertained that endogenous molecular chaperones are not induced even when IBT-30 is applied to cells. This clearly demonstrates that IBT-30 assisted in the molecular chaperone function to reduce the number of mutant protein aggregates.

### Industrial Applicability

Compounds of the present invention indicated by General Formula (1) have extremely strong chemical chaperone activity, and effectively protect cells from cell damage. Therefore, the compounds are useful for preventing, improving, or treating neurodegenerative diseases such as Alzheimer's disease, Parkinson's disease, amyotrophic lateral sclerosis (ALS), polyglutamine diseases (including Huntington's disease), Creutzfeldt-Jakob disease, and central diabetes insipidus (familial neurogenic diabetes insipidus (FNDI), secondary diabetes insipidus, idiopathic diabetes insipidus); metabolic diseases such as diabetes mellitus (type 1 diabetes mellitus, type 2 diabetes mellitus), obesity, dyslipidemia, and steatohepatitis; cardiovascular diseases such as arteriosclerosis, myocardial infarction, cerebral infarction, and high blood pressure; cancers; renal disorders; osteodystrophy; cartilage tissue diseases; and autoimmune diseases, which are diseases caused by endoplasmic reticulum stress.

## Claims

1. A compound represented by General Formula (1). 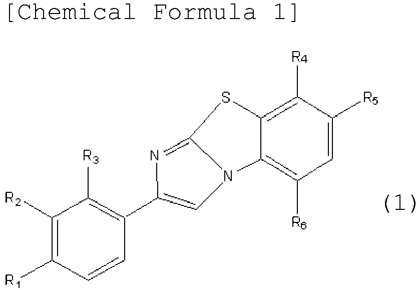
In General Formula (1), R₁ represents a hydrogen atom, a halogen atom, a lower alkyl group, a halogen-substituted lower alkyl group, a lower alkoxy group, a functional group represented by following General Formula (2), or a functional group represented by following General Formula (3), 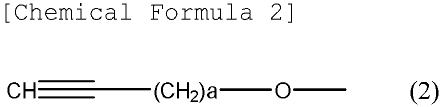 (In Formula (2), a represents an integer of 1-6.) 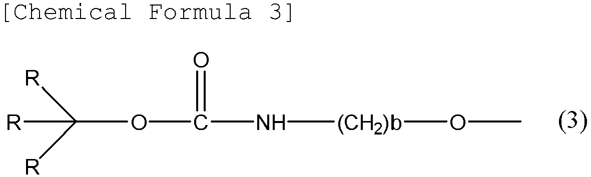 (In Formula (3), b represents an integer of 1-6, and Rs independently represent an alkyl group having 1-6 carbon atoms.)
R₂ represents a hydrogen atom, a halogen atom, a lower alkoxy group, a functional group represented by General Formula (2) above, a functional group represented by following General Formula (4), or a functional group represented by following General Formula (5), 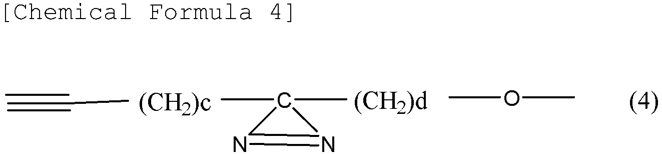 (In Formula (4), c and d independently represent an integer of 1-6.) 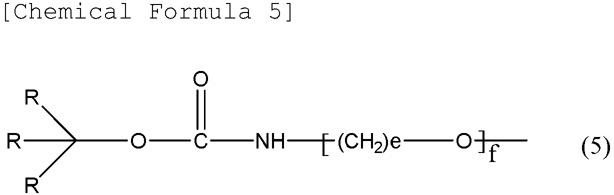 (In Formula (5), e and f independently represent an integer of 1-6, and Rs independently represent an alkyl group having 1-6 carbon atoms.)
R₃ represents a hydrogen atom or a halogen atom,
R₄ represents a hydrogen atom, a halogen atom, a functional group of following General Formula (6), or a functional group of following General Formula (7), 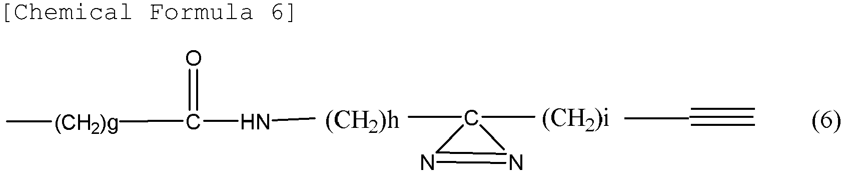 (In Formula (6), g, h, and i independently represent an integer of 1-6.) 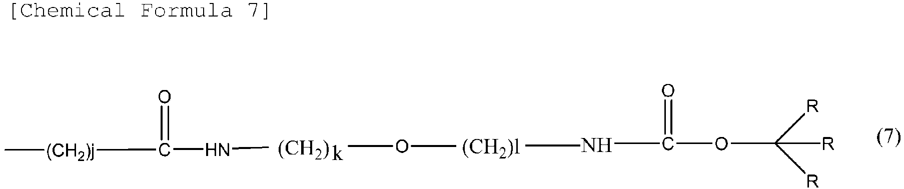 (In Formula (7), j, k, and l independently represent an integer of 1-6, and Rs independently represent an alkyl group having 1-6 carbon atoms.)
R₅ represents a hydrogen atom, a lower alkyl group, a lower alkoxy group, a hydroxy-substituted lower alkyl group, a lower alkyl-substituted sulfonyl group, a lower alkoxy-substituted carbonyl group, a functional group represented by following General Formula (8), a functional group represented by General Formula (9), a functional group represented by General Formula (10), a functional group represented by General Formula (11), a functional group represented by General Formula (12), a functional group represented by General Formula (13), or a functional group represented by General Formula (14), 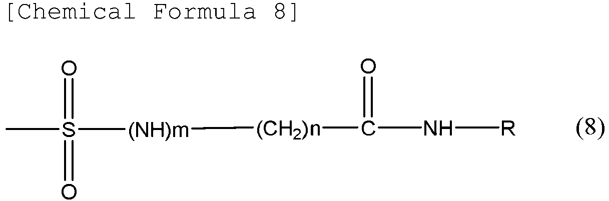 (In Formula (8), m and n independently represent an integer of 1-6, and Rs independently represent an alkyl group having 1-6 carbon atoms.) 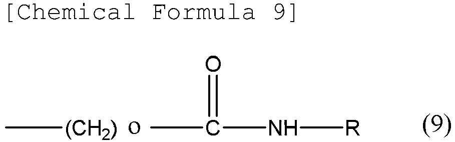 (In Formula (9), o represents an integer of 1-6, and R represents an alkyl group having 1-6 carbon atoms.) 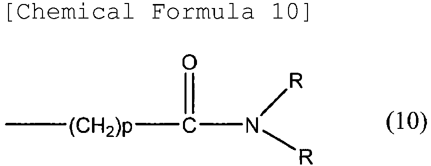 (In Formula (10), p represents an integer of 1-6, and Rs independently represent an alkyl group having 1-6 carbon atoms.) 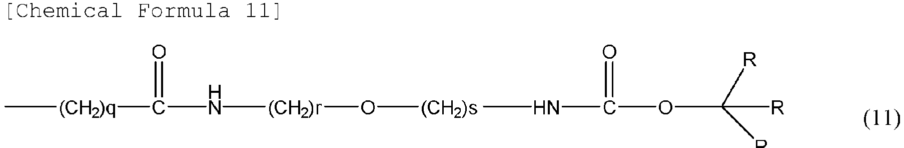 (In Formula (11), q, r, and s independently represent an integer of 1-6, and Rs independently represent an alkyl group having 1-6 carbon atoms.) 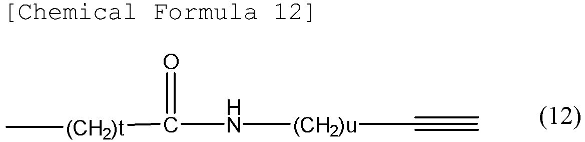 (In Formula (12), t and u independently represent an integer of 1-6.) 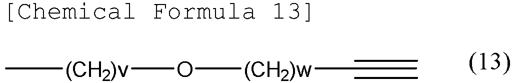 (In Formula (13), v and w independently represent an integer of 1-6.) 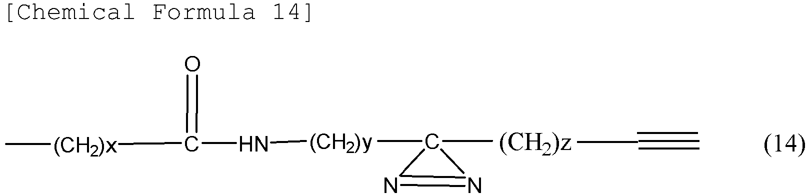 (In Formula (14), x, y, and z independently represent an integer of 1-6.)
R₆ represents a hydrogen atom or a lower alkoxy group.

2. The compound according to claim 1, wherein R_{₁} is a hydrogen atom, a halogen atom (particularly a chlorine atom), a lower alkyl group (particularly a propyl group), a lower alkoxy group (particularly a methoxy group), a halogen-substituted lower alkyl group (particularly a fluorine-substituted lower alkyl group, specifically a trifluoromethyl group), or a functional group represented by General Formula (2) (particularly a functional group of General Formula (2) in which a is 1),
R₂ is a hydrogen atom, a halogen atom (particularly a chlorine atom), a lower alkoxy group (particularly a methoxy group), a functional group represented by General Formula (2) (particularly a functional group of General Formula (2) in which a is 1), or a functional group represented by General Formula (4) (particularly a functional group of General Formula (4) in which c and d are each 2),
R₃ is a hydrogen atom or a chlorine atom,
R₄ is a hydrogen atom,
R₅ is a hydrogen atom, a lower alkyl group (particularly a methyl group), a lower alkoxy group (particularly an ethoxy group), a hydroxy-substituted lower alkyl group (particularly a hydroxy-substituted methyl group), a lower alkyl-substituted sulfonyl group (particularly a methyl-substituted sulfonyl group), a lower alkoxy-substituted carbonyl group (particularly an ethoxy-substituted carbonyl group), a functional group represented by General Formula (8) (particularly a functional group of General Formula (8) in which m is 0 or 1, n is 3, and R is a methyl group), a functional group represented by General Formula (9) (particularly a functional group of General Formula (9) in which o is 2, and R is a methyl group), or a functional group represented by General Formula (10) (particularly a functional group of General Formula (10) in which p is 2, and two Rs are each an ethyl group), and
R₆ is a hydrogen atom or a methoxy group.

3. The compound according to any one of claims 1 to 3,
wherein R₁ is a halogen-substituted lower alkyl group, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a lower alkyl-substituted sulfonyl group, and R₆ is a hydrogen atom;
a compound in which R₁ is a halogen atom, R₂ is a halogen atom, R₃ is a hydrogen atom, R₄ is a hydrogen atom, R₅ is a lower alkyl-substituted sulfonyl group, and R₆ is a hydrogen atom;
a compound in which R₁ is a hydrogen atom, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a functional group of General Formula (9) in which o is 2, and R is a methyl group,
and R₆ is a hydrogen atom;
a compound in which R₁ is a halogen atom, R₂ is a lower alkoxy group, R₃ is a hydrogen atom, R₄ is a hydrogen atom, R₅ is a lower alkyl-substituted sulfonyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (9) in which o is 2, and R is a methyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (8) in which m is 0, n is 3, and R is a methyl group, and R₆ is a hydrogen atom;
a compound in which R₁ is a halogen-substituted lower alkyl group,
R₂ is a hydrogen atom, R₃ is a hydrogen atom, R₄ is a hydrogen atom,
R₅ is a functional group of General Formula (10) in which p is 2, and two Rs are each an ethyl group, and R₆ is a hydrogen atom;
a compound in which R₁ is a halogen-substituted lower alkyl group,
R₂ is a hydrogen atom, R₃ is a hydrogen atom, R₄ is a hydrogen atom, R₅ is a hydrogen atom, and R₆ is a hydrogen atom;
a compound in which R₁ is a functional group of General Formula (2) in which a is 1, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a lower alkoxy group, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a halogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkoxy-substituted carbonyl group, and R₆ is a hydrogen atom;
a compound in which R₁ is a halogen atom, R₂ is a hydrogen atom, R₃ is a hydrogen atom, R₄ is a hydrogen atom, R₅ is a lower alkoxy group, and R₆ is a hydrogen atom;
a compound in which R₁ is a lower alkyl-substituted sulfonyl group,
R₂ is a hydrogen atom, R₃ is a hydrogen atom, R₄ is a hydrogen atom,
R₅ is a functional group of General Formula (8) in which m is 1, n is 3, and R is a methyl group, and R₆ is a hydrogen atom;
a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a hydrogen atom, R₃ is a hydrogen atom, R₄ is a hydrogen atom, R₅ is a hydroxy-substituted lower alkyl group, and R₆ is a hydrogen atom;
a compound in which R₁ is a halogen atom, R₂ is a functional group of General Formula (2) in which a is 1, R₃ is a hydrogen atom, R₄ is a hydrogen atom, R₅ is a lower alkyl-substituted sulfonyl group, and R₆ is a hydrogen atom;
a compound in which R₁ is a halogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom;
a compound in which R₁ is a lower alkyl group, R₂ is a hydrogen atom, R₃ is a hydrogen atom, R₄ is a hydrogen atom, R₅ is a lower alkyl-substituted sulfonyl group, and R₆ is a hydrogen atom;
a compound in which R₁ is a lower alkyl-substituted sulfonyl group,
R₂ is a hydrogen atom, R₃ is a hydrogen atom, R₄ is a hydrogen atom, R₅ is a lower alkyl group, and R₆ is a hydrogen atom; or
a compound in which R₁ is a hydrogen atom, R₂ is a functional group of General Formula (4) in which c and d are each 2, R₃ is a hydrogen atom, R₄ is a hydrogen atom, R₅ is a lower alkyl-substituted sulfonyl group, and R₆ is a hydrogen atom.

4. The compound according to any one of claims 1 to 3,
wherein R₁ is a hydrogen atom, a fluorine atom, a chlorine atom, a propyl group (particularly, an n-propyl group), a trifluoromethyl group, a methoxy group, an ethoxy group, a functional group of General Formula (2) in which a is 1, or a functional group of General Formula (3) in which b is 3 and the three Rs are methyl group;
R₂ is a hydrogen atom, a chlorine atom, a fluorine atom, a methoxy group, a functional group of General Formula (2) in which a is 1, a functional group of General Formula (4) in which c and d are each 2, a functional group of General Formula (5) in which e is 2, f is 1, and three Rs are methyl group, or a functional group of General Formula (5) in which e is 2, f is 3, and three Rs are methyl group;
R₃ is a hydrogen atom or a chlorine atom;
R₄ is a hydrogen atom, a bromine atom, a functional group of General Formula (6) in which g, h, and i are each 2, or a functional group of General Formula (7) in which j, k, and l are each 2 and three Rs are methyl group;
R₅ is a hydrogen atom, a methyl group, a methoxy group, a hydroxymethyl group, a methylsulfonyl group, a lower alkyl-substituted oxycarbonyl group, a functional group of General Formula (8) in which m is 0, n is 3, and R is a methyl group, a functional group of General Formula (8) in which m is 1, n is 3, and R is a methyl group, a functional group of General Formula (9) in which o is 2 and R is a methyl group, a functional group of General Formula (10) in which p is 1 and two R groups are ethyl group, a functional group of General Formula (11) in which q, r, and s are each 2 and three Rs are methyl group, a functional group of General Formula (12) in which t and u are each 2, a functional group of General Formula (13) in which v and w are each 2, or a functional group of General Formula (14) in which x, y, and z are each 2;
R₆ is a hydrogen atom or a methoxy group.

5. A chemical chaperone comprising the compound according to any one of claims 1 to 4.

6. A cytoprotectant against endoplasmic reticulum stress comprising the compound according to any one of claims 1 to 4.

7. An agent for alleviating or reducing endoplasmic reticulum stress comprising the compound according to any one of claims 1 to 4.

8. an agent for preventing, improving, or treating a disease caused by endoplasmic reticulum stress comprising the compound accord ing to any one of claims 1 to 4.

9. The agent according to claim 8, wherein the disease caused by endoplasmic reticulum stress is neurodegenerative diseases, diabetes mellitus, metabolic diseases, cardiovascular diseases, cancers, renal disorders, osteodystrophy, cartilage tissue diseases, or autoimmune diseases.

10. The agent according to claim 8, wherein the disease caused by endoplasmic reticulum stress is central diabetes insipidus.
